# EUROPEAN PATENT SPECIFICATION

(11) **EP 1 112 495 B1**
(45) Date of publication and mention of the grant of the patent: **13.08.2003**
(21) Application number: 99923760.5
(22) Date of filing: 26.05.1999
(51) Int. Cl.: C07H 3/04, C07K 1/00, A61K 31/70, C12N 15/06, C12N 1/20, A61K 39/085, C07K 16/12

(54) **DISACCHARIDE DERIVATIVES**
DISACCHARIDE-DERIVATE
DERIVES DE DISACCHARIDES

(30) Priority: 28.05.1998 GB 9811347
(43) Date of publication of application: 04.07.2001
(73) Proprietor: Oxoid Limited, Basingstoke, Hampshire RG24 8PW (GB)
(72) Inventor: LAMBERT, Peter, Anthony, Sutton Coldfield, West Midlands B74 4NR (GB); ELLIOTT, Thomas Stuart J., Sutton Coldfield, Birmingham B74 3AE (GB)
(74) Representative: Lipscombe, Martin John
(86) International application number: GB9901650
(87) International publication number: WO99061913

(56) References cited:
- WO-A-97/42343
- J.J.OLTVOORT ET AL.: "Synthesis of a Lipoteichoic Acid-Carrier Fragment of Staphylococcus aureus." CARBOHYDRATE RESEARCH., vol. 130, 1984, pages 147-163, XP002120748 ELSEVIER SCIENTIFIC PUBLISHING COMPANY. AMSTERDAM., NL ISSN: 0008-6215
- W.FISCHER: "Molecular Analysis of Lipid Macroamphiphiles by Hydrophobic Interaction Chromatography Exemplified with Lipotechoic Acids." ANALYTICAL CHEMISTRY, vol. 208, 1993, pages 49-56, XP002120749 cited in the application
- H.I.WERGELAND ET AL.: "Antibodies to Staphylococcal Peptidoglycan and its Peptide Epitopes, Techoic Acid, and Lipotechoic Acid in Sera from Blood Donors and Patients with Staphylococcal Infections." JOURNAL OF CLINICAL MICROBIOLOGY, vol. 27, no. 6, June 1989 (1989-06), pages 1286-1291, XP002120750 cited in the application
- J.J.OLTVOORT ET AL.: "A Simple Approach to the Synthesis of a Membrane Techoic Acid Fragment of Staphylococcus aureus." RECUEIL, JOURNAL OF THE ROYAL NETHERLANDS CHEMICAL SOCIETY, vol. 101, no. 3, March 1982 (1982-03), pages 87-91, XP002120751
- DATABASE WPI Section Ch, Week 198703 Derwent Publications Ltd., London, GB; Class B04, AN 1987-017786 XP002120752 & JP 61 275217 A (YAKULT HONSHA KK), 5 December 1986 (1986-12-05)

## Description

### Field of the Invention

The present invention relates, *inter alia,* to a novel bacterial antigen, compositions and kits for use in the diagnosis of bacterial infections, and to a method of diagnosing a bacterial infection, and to a method of preventing and/or treating a bacterial infection.

### Background of the Invention

Most bacteria can be classified into one of two groups, Gram positive (+ve) or Gram negative (-ve), depending on the way in which the bacterial cells react with Gram stain, which is in turn dependent on the composition of the bacterial envelope. Gram positive bacteria include many important pathogens of humans and animals. Examples include *Staphylococcus spp.,* such as *Staph. aureus*, and *Streptococcus spp.* such as *Strep. pyogenes.*

The envelopes of Gram +ve bacteria differ between genera, and between species within a single genus. However, the typical Gram +ve bacterial envelope comprises a lipid cell membrane, surrounded by a relatively rigid shell of peptidoglycan. In addition to phospholipids and glycolipids, the lipid membrane also comprises lipoteichoic acids. Lipoteichoic acid (LTA) has been extensively studied and detailed reviews have been provided by Fischer ("Physiology of Lipoteichoic acids in Bacteria" in Advances in Microbial Physiology 29, 233-302, 1988; and Med. Microbiol. Immunol. 183, 61-76, 1994).

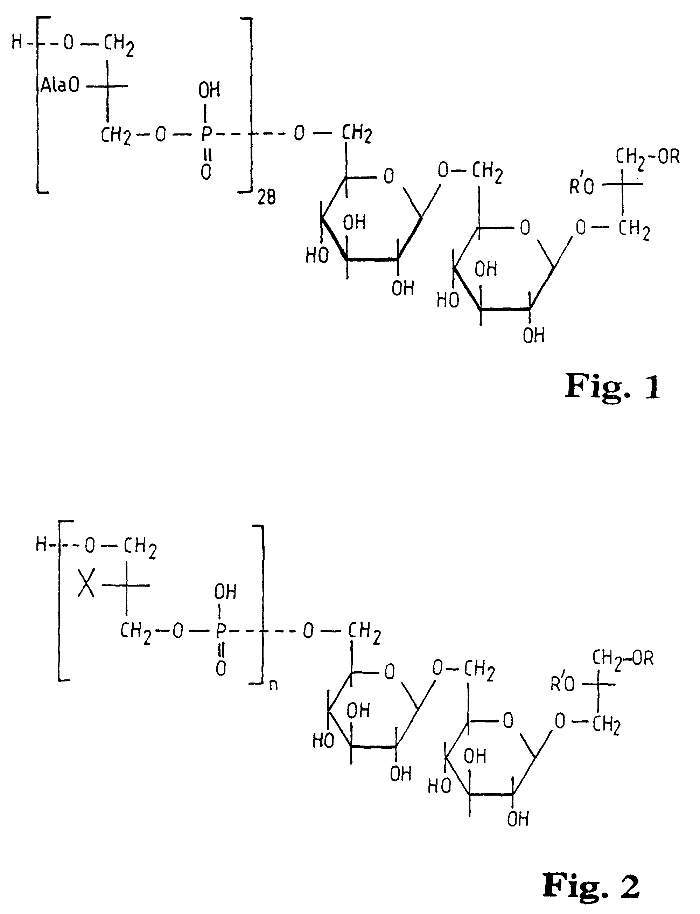

In essence, LTA comprises a hydrophilic 1,3-linked polyglycerophosphate chain covalently linked to a hydrophobic glycolipid. For *Staph. aureus*, the average LTA chain contains about 25 glycerophosphate residues, of which between 30 and 80% (typically about 70%) are generally substituted with a D-alanyl residue at position 2, and around 10% are substituted with N-acetyl glucosamine residues. The typical structure of a conventional LTA molecule is illustrated schematically in Figure 1 for reference. LTA is usually located in the bacterial membrane, but is also secreted into the extracellular medium during growth.

LTA is known to be antigenic in humans suggesting that, in theory, diagnosis of infection by Gram +ve bacteria might be possible by serological methods aimed at detection of anti-LTA antibodies in the sera of patients. However, a previous study by Wergeland *et al*, (Journal of Clinical Microbiol. 27, 1286-1291, 1989) found that "the predictive values of these staphylococcal antibodies were too low to be of diagnostic value" (i.e. the antibody titres of controls and infected subjects could not be satisfactorily discriminated, because healthy uninfected individuals also possess antibodies to LTA).

Gram +ve bacteria cause a number of infections, especially nosocomial or iatrogenic infections which currently may be extremely difficult to diagnose. Examples include infections associated with central venous catheters (CVC), bone infections (especially hip replacements), continuous ambulatory peritoneal dialysis (CAPD), bacterial endocarditis, and cerebrospinal fluid shunts. Accordingly, a serodiagnostic test for detecting Gram +ve infections, especially in the instances referred to above, would be highly desirable, but at present no suitable test is available and diagnosis relies instead on the ability to isolate infecting organisms in culture. Such culture tests are very slow and can be unreliable if the patient has received antibiotic treatment.

Oltvoort *et al* (1984 Carbohydrate Research 130, 147-163) describe the chemical synthesis of a lipoteichoic acid carrier fragment comprising three glycerol phosphate units (i.e. a compound wherein n = 3 and x = OH in Figure 2 of the present specification). The authors state that "the antigenic properties of membrane teichoic acids are not of primary physiological significance" and were interested instead in the role of lipoteichoic acids as a carrier molecule in the biosynthesis of (bacterial) wall teichoic acids.

### Summary of the Invention

In a first aspect, the invention provides an isolated compound having the structure 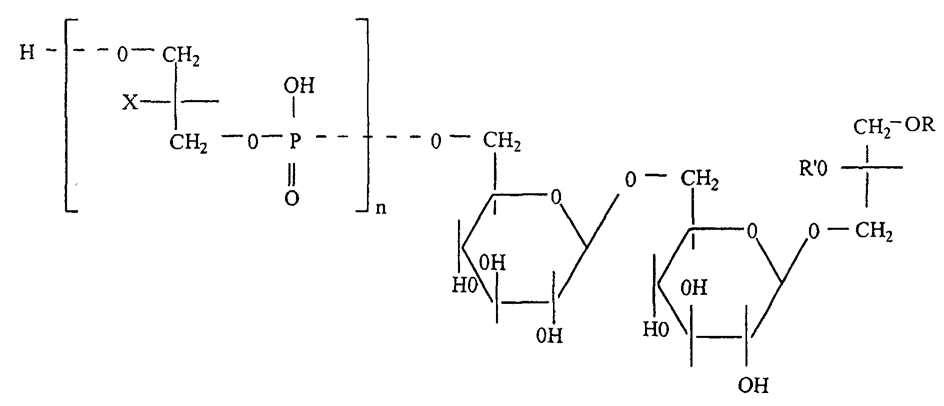 wherein n is an integer between 3 and 10 inclusive and X is H, OH, alkyl, aryl, amyl, or an amino acid residue, optionally substituted, or a sugar residue, optionally substituted, and wherein R and R¹ are hydrophobic hydrocarbon or fatty acid chains and wherein R may be the same as R¹ or different, and wherein the value of n in any single sample is a single integer, other than a compound wherein n is 3 and X is OH.

The term "isolated", with respect to the compound of the invention, is intended to indicate that whilst n may vary from 3 to 10 (inclusive) for compounds within the scope of the invention, the value of n in any single sample of the isolated compound is a single integer. Thus, whilst a short chain LTA preparation has been provided in the prior art (Fischer 1993 Analytical Biochemistry 208, 49-56), the prior art preparation comprised fractions of mixed chain lengths (i.e. different values of n).

Typically, n will have a value between 4 and 8 (inclusive) and desirably n = 6. Preferably X = H, OH, D-alanyl or N-acetyl glucosamine. It will be apparent to those skilled in the art that in the structure represented in Figure 2, the identity of X may vary from repeat to repeat so that, for example, where n = 6, X may be H in 4 repeat units, D-alanyl in one unit, and N-acetyl glucosamine in another unit.

In a second aspect the invention provides a composition in which, of the bacterial components present, at least 50 w/w % comprises a compound having the structure 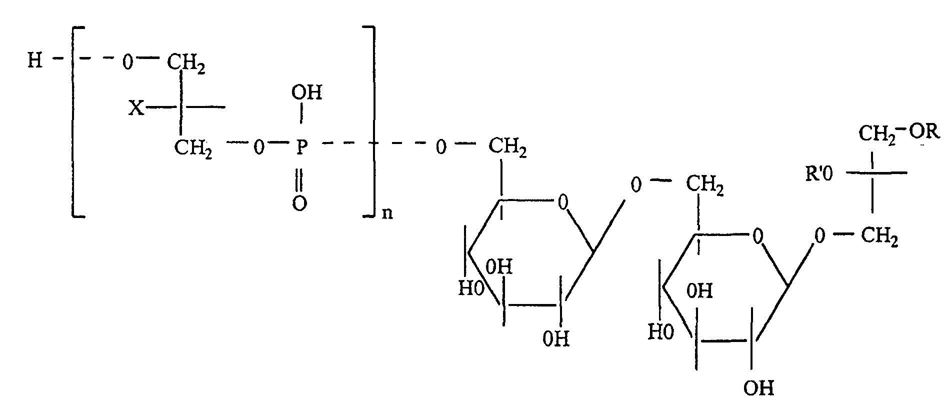 wherein n is an integer between 3 and 10 inclusive and X is H, OH, alkyl, aryl, amyl, or an amino acid residue, optionally substituted, or a sugar residue, optionally substituted, and wherein R and R¹ are hydrophobic hydrocarbon or fatty acid chains and wherein R may be the same as R¹ or different, other than a compound wherein n is 3 and X is OH.

The term "substantially pure", as used herein with reference to the composition of the second aspect of the invention is intended to indicate that, of the bacterial components present in the composition, the compound having a structural formula in accordance with Figure 2 comprises at least 50 w/w %, preferably at least 70 w/w %, and most preferably at least 80 w/w %. Those skilled in the art will appreciate that the composition may comprise other solids, which are not bacterial components, in greater amounts such as salts, buffers and inert carrier substances. The composition may take any convenient form e.g. freeze dried solid, aqueous solution, immobilised on a solid support (e.g. as a test kit component or the like). The composition may typically comprise an isolated compound in accordance with the first aspect of the invention.

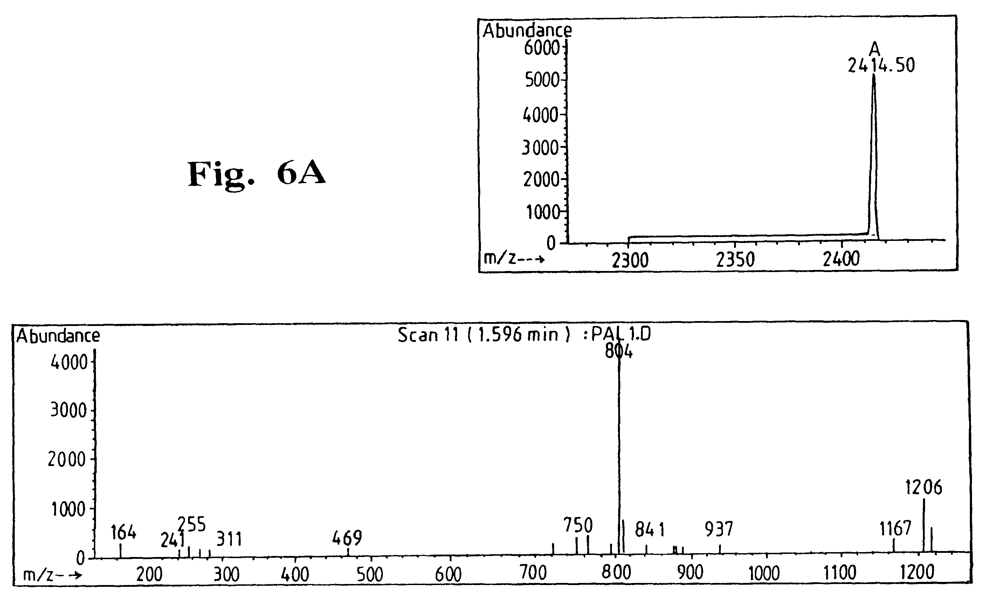

Where the composition comprises a protein component (especially a bacterial-derived protein component) it is preferred that this is present in a non-denatured form. In particular embodiments, the composition may give rise to a negative electrospray mass spectrum of the character shown in Figures 6A-6C, with a pronounced peak at an m/z ratio of 2414.

The inventors have found that compositions in accordance with the invention can act as antigens which unexpectedly are able to form the basis of an immunological test for infection of a subject by a Gram +ve bacterium.

Accordingly, in a third aspect, the invention provides a method of testing for a Gram +ve bacterial infection in a mammalian, typically human subject, the method comprising the steps of: contacting a sample of body fluid from the subject with a composition comprising a compound having the structure 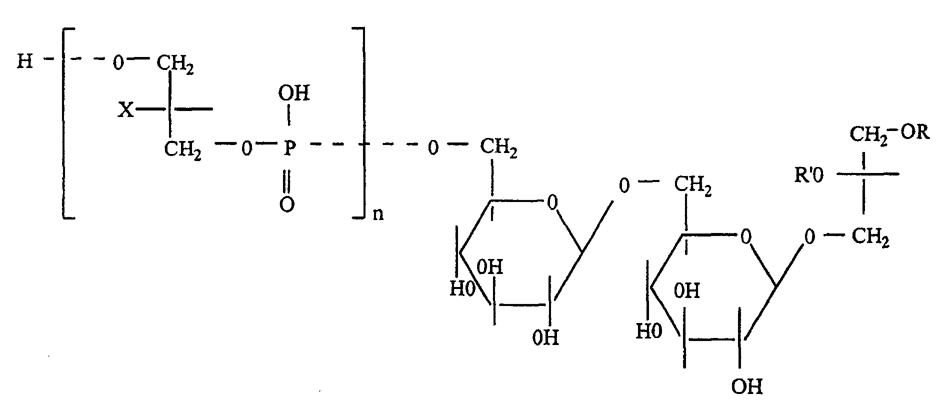 wherein n is an integer between 3 and 10 inclusive and X is H, OH, alkyl, aryl, amyl, or an amino acid residue, optionally substituted, or a sugar residue optionally substituted, and R and R¹ are hydrophobic hydrocarbon or fatty acid chains wherein R may be the same as R¹, or different; and detecting binding of antibodies, if any, in the sample to the composition. Preferably the sample of body fluid from the patient will be a blood or serum sample, although other fluids (such as saliva, cerebrospinal fluid, or urine) may also be useful. Conveniently, a blood sample from a subject will be treated (e.g. by centrifugation) to provide a serum sample for use in the method defined above. Desirably the method will comprise detection of IgG and/or IgM antibodies produced by the patient in response to infection.

Conveniently, the composition of the second aspect of the invention is of use in performing the method of the third aspect, such that the composition may advantageously be provided in immobilised form, bound to a solid surface such as a microscope slide, test card, capillary fill chamber, or the wells of a microtitre plate, or on the surface of a latex bead or other particulate support. Methods of immobilising materials to solid supports are well known to those skilled in the art and include utilisation of either specific or non-specific interactions, including elecrostatic or hydrophobic interactions, absorption, covalent bonding, and the like.

Numerous assay formats suitable for performing the method of the invention will readily be apparent. Particularly desirable and convenient is an enzyme-linked immunosorbent assay (ELISA) format, in which binding of antibodies in the sample to the composition is detected by binding of an enzyme-labelled second antibody, which is then monitored by the amount of coloured product produced upon the addition of an appropriate substrate. Other suitable assay formats include radioimmunoassay (RIA), Western blots, and so-called "Rapid Assay" techniques.

The method of the invention is believed to be suitable for testing for infection caused by the majority of Gram +ve organisms, especially Gram +ve cocci such as *Streptococcus spp.* (especially *Streptococcus pyogenes* and viridans streptococci) *Staphylococcus spp.* such as *Staph. aureus* (but especially coagulase negative staphylococci ["CNS"], such as *Staph. epidermidis*), *Enterococcus faecalis*, and *Enterococcus faecium.*

The method will be particularly useful in diagnosing the presence of infection associated with central venous catheters, CAPD, prosthetic devices (e.g. replacement hips or knees), cerebrospinal fluid shunts, bone infections (e.g. osteomyelitis, discitis) or endocarditis.

In a fourth aspect, the invention provides a diagnostic test kit for diagnosing Gram +ve infections in a mammalian subject, the kit comprising: a solid support for performing the test; and a composition in accordance with the second aspect of the invention. Desirably the composition may be covalently coupled to the solid support, or may be immobilised thereon in some other way (e.g. by absorption). Alternatively, the composition may be provided in the kit as a solution, suspension or dry powder. The solid support conveniently takes the form of a conventional assay component, such as a microtitre plate of synthetic plastics material, or a microscope slide, or a test surface comprising card or a synthetic laminate material.

The kit will preferably comprise one or more of a number of other components of a generally conventional nature, such as detection reagents (e.g. labelled antibodies, enzyme substrates or other substances for developing a colour), positive and negative control samples, instructions for use and so on.

In a fifth aspect, the invention provides a sterile composition for use as a vaccine in a mammalian subject; the vaccine comprising a composition in accordance with the second aspect of the invention. Conveniently, the vaccine is provided in unitary dose form. The size of the dose will depend on the identity of the mammalian subject. For example, large animals such as cattle or horses may be given doses which comprise 100mg to 1 gram of a substance having the structure shown in Figure 2.

In comparison, an efficacious dose (i.e. one which induces a detectable immune response in the subject) for humans might be in the range of 10-500mgs. It will be apparent that repeat doses of the vaccine composition may be administered until the subject has developed a detectable immune response. A convenient means of detecting an immune response is to determine the presence of serum antibodies directed against the vaccine antigen, which test may be performed substantially as outlined above. Typically the vaccine will be provided as a solution for injection. Such solutions will conveniently comprise a composition in accordance with the second aspect in which the solvent takes the form of sterile water or a sterile aqueous solution such as saline or phosphate-buffered saline. The vaccine of the invention may be given, for example, intra-muscularly intra-peritoneally, sub-cutaneously or intra-venously. Other routes of administration (e.g. intranasal, oral) may also be possible.

For maximum efficacy, the vaccine may additionally comprise other bacterial antigens, especially those from Gram +ve bacteria, which are known to be the target of an immune response. Additionally the vaccine may comprise adjuvant materials, such as alum, or complete or incomplete Freund's adjuvant, immunostimulatory complexes, liposomes and the like.

The vaccine will have the effect of protecting the recipient subject against infection by one or more Gram +ve bacteria. This protection may be total (i.e. prevent the subject from developing any symptoms following exposure to a Gram +ve pathogen) or partial (i.e. lessen the severity of disease, to a greater or lesser extent, following exposure to the pathogen).

In a sixth aspect, the invention provides a method of obtaining an immunoglobulin or antigen-binding variant thereof having specific binding for a compound in accordance with the first aspect of the invention, the method comprising the steps of: screening a library of viruses or other particles displaying an immunoglobulin or antigen-binding variant thereof on their surface; and selecting those members of the library which display an immunoglobulin or antigen-binding variant thereof which bind to the compound.

In a preferred embodiment, the method provides a method of making monoclonal antibodies.

A suitable method of obtaining antibodies to the compound shown in Figure 2 is disclosed for example in WO 92/01047. Such screening methods are also now well-known to those skilled in the art.

Immunoglobulins having specific binding activity for the compound shown in Figure 2 could be useful in a number of ways. The immunoglobulins could be conventional antibodies (e.g. IgG, IgA, IgM etc) or may be variants thereof, such as Fab, Fv scFv, bispecific antibody, or may be chimeric proteins comprising antigen-binding portions of antibody molecules. Methods of preparing such variants are all well-known to those skilled in the art. Such molecules may be useful in diagnostic kits (e.g. as positive control samples), or may be useful in the passive immunisation of a mammalian subject for the prevention and/or treatment of a Gram +ve infection, and a method of preventing and/or treating a Gram +ve infection in a mammalian subject in such a way constitutes a further aspect of the invention. The invention thus also provides a composition comprising an immunoglobulin, or antigen-binding variant thereof, having specific binding ability for a compound in accordance with the first aspect of the invention. The immunoglobulin will preferably be in substantially pure form.

In a seventh aspect, the invention provides a method of making a composition in accordance with the second aspect, the method comprising the steps of: culturing a Gram +ve bacterium in a growth medium so as to cause the bacterium to secrete into the growth medium a compound in accordance with the first aspect of the invention; separating the growth medium from the bacterial cells; fractionating the growth medium; and isolating that fraction which comprises, in substantially pure form, the compound in accordance with the first aspect.

Suitable growth conditions for the bacterium will be apparent to those skilled in the art. Typically these will comprise incubation at, or about, 37°C with agitation. The growth medium may be a complex medium (e.g. brain heart infusion or BHI), or a chemically defined medium such as HHW, detailed in the examples below (which may simplify fractionation and purification of the desired compound). Following a suitable period of growth (e.g. 18-36 hours), during which the compound having the structure shown in Figure 2 is released from the bacterial cells into the medium, the growth medium may be separated from the cells by passage of the culture through a filtration means (the mean pore diameter of which is small enough to retain the bacterial cells) or, more preferably by centrifugation. The culture may be subjected to centrifugation in batches, or continuously by passage through a continuous flow centrifuge.

Conveniently, once the cells have been removed, the medium is concentrated. Desirably concentration is achieved by freeze drying and redissolving/resuspending in a reduced volume of fluid (such as distilled water). Conveniently a 5-20 times concentration (typically 10X) may be effected. Following optional concentration, the medium is fractionated.

A preferred method of fractionation is to contact the medium with a chromatographic separation means. A large number of chromatographic separation means are known including ion-exchange chromatography. Desirably the chromatographic separation means comprises gel permeation chromatography. Conveniently a suitable gel permeation chromatography resin is packed in a column, and the medium allowed to pass through the column. One resin which the inventors have found particularly suitable is Superose 12 (available from Amersham Pharmacia), but a large number of similar resins are available and which could be suitable for the desired purpose.

A large number of Gram +ve organisms may be suitable for use in the method of preparing the composition. Conveniently the organism is not an obligate anaerobe. Preferred organisms are Gram +ve cocci, such as Streptococci or Staphylococci. More particularly, coagulase negative staphylococci (CNS) are preferred, which may be slime-producing strains or non-slime producing. Conveniently *Staphylococcus epidermidis* may be used.

The inventors have found that a number of strains isolated from patients are particularly useful in preparing the composition of the first aspect of the invention. These strains have all been made the subject of a deposit (under the terms of the Budapest Treaty) at the National Collections of Industrial and Marine Bacteria (NCIMB, 23 St Machar Drive, Aberdeen AB2 1RY, United Kingdom), as detailed below:
Strain 1 CAN 6KIII (Accession No. NCIMB 40896, date of deposit 18.9.97)
Strain 2 HAR 6KIV (Accession No. NCIMB 40945, date of deposit 22.4.98)
Strain 3 COS 6KV (Accession No. NCIMB 40946, date of deposit 22.4.98)
Strain 4 MIL 6LI (Accession No. NCIMB 40947, date of deposit 22.4.98)
Strain 5 HED 6LIII (Accession No. NCIMB 40948, date of deposit 22.4.98)
Strain 6 ONE 6KVI (Accession No. NCIMB 40949, date of deposit 22.4.98)
Strain 7 MAT 6LII (Accession No. NCIMB 40950, date of deposit 22.4.98)

Strains 1-5 are strains of *Staph. epidermidis.* Strain 6 is a strain of *Staph. haemolyticus* and strain 7 is a *Micrococcus kristinae* strain. Strains 1, 3, 5 and 7 are slime-producing strains, and strains 2, 4 and 6 are non-slime-producing, as judged by investigation on Congo Red medium (described in Example 1 below).

The deposits were made by the present inventors acting for and on behalf of the Applicant.

One particular strain, or (preferably) any combination of the above strains, may be used for preparation of a composition in accordance with the second aspect of the invention.

The inventors have found that it may be preferable to prepare the composition of the invention by pooling growth medium obtained from culturing several different organisms, either separately or (less preferably) in a mixed culture. The inventors have found that a composition prepared in such a way has unexpectedly superior antigenic properties. In particular, the invention provides a composition obtained by pooling the growth medium of cultures of any two or more of strains 1-7 (having the accession numbers identified above); separating the growth medium from the cells; and fractionating the medium as defined above.

The invention will now be further described by way of illustrative example and with reference to the accompanying drawings, in which;
Figure 1 shows the chemical structure of conventional "long chain" lipoteichoic acid molecules;
Figure 2 shows the structure of a short chain compound present in compositions of the present invention;
Figure 3 is a graph of absorbance against fraction number, showing an elution profile;
Figures 4A and 4B are scatter graphs showing IgG titre (Fig. 4A) or IgM titre (Fig. 4B) as measured by BHI7 ELISA for patients with CVC sepsis (left hand plot) or uninfected control patients (right hand plot);
Figure 5 is a graph of true positive rate against false positive rate, showing ROC (Receiver Operator Characteristic) analysis of different methods for diagnosis of CVC sepsis;
Figures 6A-6C show negative electrospray mass spectra as plots of abundance against mass/charge ratio (m/z) (lower frames) and deconvolution plots of these data (upper frames), for strain 1 (6A), strain 3 (6B) and strain 4 (6C); and
Figure 7 is scattergraph showing antibody titres of sera from infected patients (lozenge symbols) or uninfected conrols (squares).

The inventors have developed a direct ELISA test to measure serum levels of IgG or IgM which react with the short chain LTA derived from strains of *Staph. epidermidis* coated onto polystyrene microtitre plates. The short chain LTA is extracted from brain heart infusion broth culture supernatants of *Staph. epidermidis* strains isolated from patients with CVC-related sepsis and partially purified by gel permeation chromatography. Trials have been conducted to evaluate the diagnostic potential of the assay and to characterise the antigen. Trials included sera from patients with infected catheters or sepsis related to the CVC, patients with endocarditis, and from control patients with CVC in place but no infection. This study has shown that the assay detects significantly higher levels of IgG and IgM in serum from the infected patients than in serum from the control patients. The test therefore offers a means of rapid indication of Gram-positive infection without the need for positive blood cultures. The test will have applications in these and other infections where positive blood cultures are difficult to obtain, especially after commencement of antibiotic therapy.

### Example 1 - Selection of antigen fractions for use in ELISA

The antigen extract used to coat the ELISA microtitre plates was identified by screening a range of cellular and exocellular fractions from *Staph. epidermidis* isolates obtained from patients with CVC-related sepsis. Twenty different strains were examined for colonial appearance on a brain heart infusion/congo red/sucrose medium to distinguish slime-producing strains from non-slime producers. The inventors found that approximately 25% of the isolates did not produce slime and therefore decided not to attempt a serodiagnostic test based exclusively on the slime component. Instead, antigenic material common to all the strains was recovered from the brain heart infusion liquid medium in which the cells had been grown and purified by gel permeation chromatography.

The strain was grown in brain heart infusion (Oxoid) for 18 h with shaking at 37°C. Cells were removed by centrifugation (10,000 g, 10 min) and the medium concentrated ten-fold by freeze drying and resuspension in water. Samples of the concentrated culture medium (1 mL) were applied to a Superose 12 10/30 FPLC column (Pharmacia), eluted with water at 0.4 ml/min, collecting 0.8 mL fractions. Fractions were assayed for hexose and protein using phenol/sulphuric acid (Dubois *et al*, 1956 Analytical Chemistry **28**, 350-356) and Lowry assays (Lowry *et al*, 1951 J. Biol. Chem. **193**, 265-275) respectively.

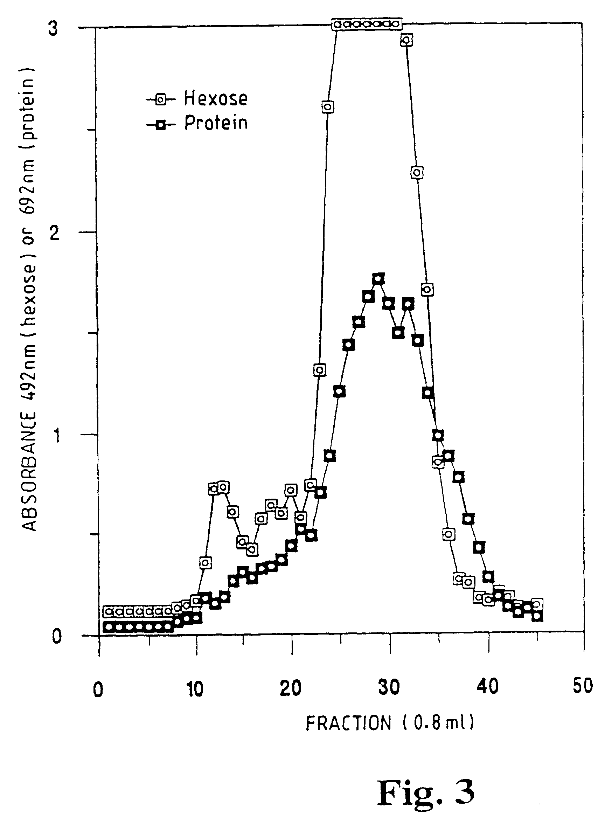

Figure 3 shows the typical elution profile of growth medium recovered from a non slime-producing *S. epidermidis* isolate. In Figure 3, the amount of hexose (determined by measuring absorbance at 492nm following colourimetric assay) is denoted by the open symbols, and the amount of protein (determined by measuring absorbance at 692nm) is denoted by the partially filled symbols. The large peaks of material in fractions 20-40 represent components of the brain heart infusion and were also present in the elution profile when fresh brain heart infusion was applied to the column (data not shown). Fractions 10-15, eluting immediately after the void volume of the column, contain some hexose but very little protein. Subsequent chemical and immunochemical studies have shown this material to be related to LTA.

Although preliminary studies were made with antigen prepared from this single strain, the inventors were concerned that this preparation might lack key antigenic determinants produced by other bacterial strains. Accordingly the number of strains used to make the antigenic composition employed in the assay was increased to seven. These seven (strains 1-7) were selected from the twenty originally studied and comprised four slime-producers and three non slime-producers. These strains have all been made the subject of a deposit under the Budapest Treaty (Accession details given above). They were chosen as including representatives of all the colonial types encountered in the study. Culture supernatants from each strain were pooled, concentrated and fractionated by FPLC as described above. Fractions 10-15 were pooled and used to coat the wells. This purified pooled antigen was designated "BHI7".

Essentially similar techniques could be used to prepare antigen on an industrial scale, simply by scaling-up the procedure. An alternative preparation procedure could be based on an adaptation of the hydrophobic interaction chromatography (HIC) method disclosed by Fischer (Analyt. Biochem. 1993, **208**, 49-56). This published method involves initial extraction from whole cells using phenol-water followed by purification by HIC on octyl-Sepharose, but as the inventors have found antigen in the growth medium, it would be preferred to apply the purification method directly to the medium by (optionally) concentrating by freeze drying, and running the medium directly down an HIC column. The antigen alone should bind and can be eluted with a linear propanol gradient in 0.05M sodium acetate buffer, pH 4.7.

### Example 2: ELISA system

A direct ELISA system for measuring IgG or IgM levels in patient serum was developed. Purified antigen (BHI7) prepared as described in Example 1 was coated onto ELISA plates which were then washed and blocked with Tween 20. The blocked plates were stored empty at -20°C until required. The wells were probed sequentially with patients' serum (at doubling dilutions), followed by protein A-peroxidase or goat anti-human IgG-peroxidase conjugates (for detection of IgG) or goat anti-human IgM-peroxidase conjugate (for detection of IgM) and developed with a chromogenic substrate (tetramethylbenzidine/ H₂O₂). The detailed protocol is as follows:

Pooled fractions 10-15 from the FPLC purification were diluted with 100 volumes of sodium carbonate/bicarbonate buffer (0.05M, pH 9.6) and used to coat microtitre plates (Immulon 2, Dynatech) at 4°C for 18h (100 µl per well). The antigen was discarded and the wells washed with TBS-Tween (0.01M Tris-HCl pH 7.4, NaCl 0.9% w/v, 0.3% v/v Tween-20) to remove residual antigen. Unbound sites in the wells were blocked by incubation in the same buffer (1 h at 4°C). 0.1 mL samples of patient sera diluted appropriately in TBS-Tween were added to the wells and incubated for 18 h at 4°C. After removal of the serum and washing with TBS-Tween, bound IgG was detected by addition of 100 µL of protein A-horseradish peroxidase conjugate (0.5 µg/mL in TBS-Tween; Sigma) for 2 h at 4°C.

After removal of the conjugate and washing with TBS-Tween, 100 µL of chromogenic substrate was added to each well. The substrate contained 10 mg of 3,3',5,5'-tetramethylbenzidine (Sigma) dissolved in 1 mL dimethyl sulphoxide and diluted into 100 mL of sodium acetate/citrate buffer (0.1M, pH 6.0) containing 10 µL of H₂O₂ (20% v/v). When the colour had developed sufficiently (5 min at 20°C) the reaction was stopped by addition of 50 µL of sulphuric acid (1M) to each well. The yellow coloured product in each well was measured at 450nm with an Anthos 2001 plate reader (Anthos Labtec Instruments).

### Example 3: Evaluation of ELISA test using patient sera

The ELISA test was evaluated using sera from the following groups of patients:
CVC-related sepsis due to *Staph. epidermidis* (n = 39)
*Staph. epidermidis* endocarditis (n = 15)
Discitis infections due to *Staph. epidermidis* (n = 14)
controls, i.e. patients with CVCs but no infection (n = 33)

The inventors used as an endpoint for titres that serum dilution which reduced the colour production in test wells to that of control wells developed with no serum. In all assays the absorbance of such control wells was in the range 0.15-0.20.

The results for IgG titres measured with protein A-peroxidase conjugate are summarised in Tables 1 and 2 below.

**Table 1:**

| **Comparison of means and standard deviations for IgG titres for infected patients and uninfected controls** | |
|---|---|
| **Patient group (n)** | **Mean IgG titre (SD)** |
| CVC-sepsis (39) | 51,600 (47,500) |
| endocarditis (15) | 58,000 (49,000) |
| discitis (14) | 60,200 (117,300) |
| controls (33) | 8,700 (4,600) |

**Table 2:**

| **Statistical analysis of IgG titres (relative to controls)** | | | |
|---|---|---|---|
| **Statistical parameter** | **CVC-sepsis** | **Endocarditis** | **Discitis** |
| mean difference | 42,900 | 49,300 | 51,500 |
| unpaired t-test | <0.0001 | 0.0017 | 0.1245 |
| two-tailed P-value | extremely significant | very significant | not significant |
| Mann-Whitney test | <0.0001 | <0.0001 | 0.0083 |
| two-tailed P-value | extremely significant | extremely significant | very significant |

The statistical tests show a significant difference in IgG levels for both the CVC-sepsis and endocarditis patients compared with the controls. For the discitis patients which show a wide range of titres, only the non-parametric Mann-Whitney test shows a significant difference compared with the controls.

To determine further the nature of the antibody response, assays were also carried out using alternative goat anti-human IgG-, and IgM- peroxidase conjugates to detect IgG and IgM. These assays were carried out at single serum dilutions of 1:800 and the results recorded as the amount of colour developed in the assay in 5 minutes. The results are shown in Tables 3 and 4. The IgG levels detected with the anti-human IgG conjugate generally matched those obtained with the protein A conjugate. The IgM responses were lower than the IgG levels.

**Table 3:**

| **Mean absorbances for positive and negative CVC sepsis sera** | | |
|---|---|---|
| Conjugate used to develop BHI 7 ELISA plates | Mean (SD) CVC- sepsis (n = 33) | Mean (SD) CVC, no infection (n = 22) |
| Protein A-HRP | 1.2620 (0.3049) | 0.6250 (0.1802) |
| Anti-human IgG-HRP | 0.7453 (0.1563) | 0.3875 (0.1178) |
| Anti-human IgM-HRP | 0.3745 (0.1907) | 0.2160 (0.1029) |

**Table 4:**

| **Statistical analysis (unpaired t-test) of ELISA results** | |
|---|---|
| Conjugate used to develop BHI 7 ELISA plates | Difference in means, CVC-sepsis and CVC, no infection |
| Protein A-HRP | 0.6732, P<0.0001 extremely significant |
| Anti-human IgG-HRP | 0.3578, P<0.0001 extremely significant |
| Anti-human IgM-HRP | 0.1585, P = 0.0002 extremely significant |

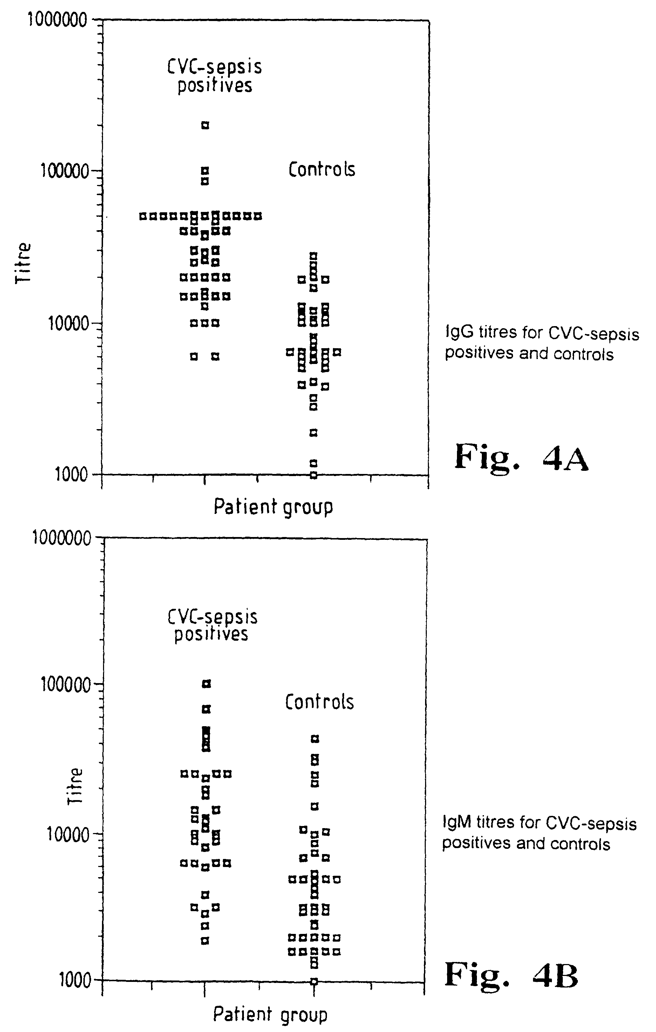

Figures 4A and 4B show scatter graphs, indicating the ELISA titres for serum IgG and IgM titres respectively for infected patients (n = 48) and the control group (n = 44). Both IgG and IgM titres show an extremely significant difference between the means of the positive and control groups. However, the scatter graphs show some overlap in the populations which is especially marked for IgM.

The diagnostic performance of the IgG and IgM ELISA tests can be compared with other methods for diagnosis of CVC-sepsis using the data and method of analysis disclosed by Siegman-Igra *et al* (Journal of Clinical Microbiology, 1997, **35,** 928-936).

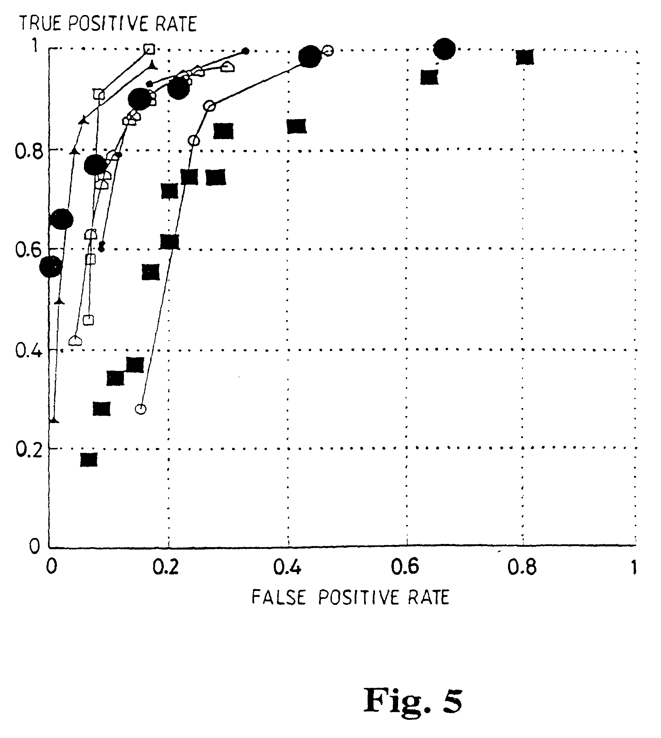

Siegman-Igra *et al* (1997) compared the different methods for diagnosis of vascular catheter related bloodstream infection by analysis of receiver-operator characteristic (ROC) curves (plots of true positive rates vs false positive rates). Summary ROC curves (calculated by pooling the published data for each method) are shown in Figure 5 using the IgG and IgM ELISA titres from Figures 4A and 4B.

In Figure 5, the different diagnostic methods are denoted as follows:

qualitative catheter segment culture (open circles); semi-quantitative catheter segment culture (open pentagons); quantitative catheter segment culture (open squares); unpaired qualitative blood culture (filled circles); and unpaired quantitative blood culture (filled triangles). The equivalent data points for the ELISA test have been plotted for IgG and IgM as large unjoined filled circles and large unjoined filled squares respectively. In essence, the closer the plots are to the top left hand corner of the graph, the more reliable is the method of diagnosis.

The results confirm that the IgG ELISA test is very effective in the diagnosis of CVC-sepsis, its performance compares favourably with the best of the published methods (unpaired quantitative blood culture).

### Example 4: Analysis of Individual Strains

The composition of the individual components of BHI7 and their contribution to the ELISA reaction was investigated by separation on sodium dodecyl sulphate polyacrylamide gel electrophoresis (SDS-PAGE). Gels were either stained with silver to reveal protein and polysaccharide constituents or were subjected to immunoblotting and reaction with patient sera. Each of the 7 strains used to prepare the BHI7 antigen was grown separately in brain heart infusion, the culture media were collected, concentrated 10-fold, purified by gel permeation chromatography and fractions 10-15 collected as for the combined BHI7 preparation.

These results (data not shown) indicated that although the antigen preparation from each component strain of BHI7 did contain some proteins (detected as sharp bands on the silver-stained gels), they were not strongly antigenic as determined by immunoblotting. The dominant antigenic components migrated as fast-moving material (i.e. low molecular weight and negatively charged) at the front of the gel forming a diffuse band of immunoreactive material on the blots. Little material was detected by silver staining of the gels in this region. This suggests that the major antigenic material is BHI7 is neither protein (which was present is small amounts but not immunoreactive) nor polysaccharide (which should be detected by silver staining and would migrate more slowly on the gels).

The individual contribution of each strain to the reactivity of BHI7 was determined by carrying out separate ELISA reactions with selected sera from CVC-sepsis, endocarditis patients and controls. The results are shown in Table 5.

**Table 5:**

| **IgG ELISA titres* using BHI7 and antigen from individual strains** | | | | | | | | |
|---|---|---|---|---|---|---|---|---|
| Serum | BHI7 | strain 1 | strain 2 | strain 3 | strain 4 | strain 5 | strain 6 | strain 7 |
| control 1 | 6,000 | 1,000 | 1,500 | 1,000 | 800 | 1,500 | 2,500 | 20,000 |
| control 2 | 3,000 | 1,000 | 500 | 800 | 800 | 700 | 1,500 | 10,000 |
| CVC-sep 1 | 100,000 | 50,000 | 50,000 | 12,000 | 40,000 | 50,000 | 40,000 | 100,000 |
| CVC-sep 2 | 30,000 | 20,000 | 12,000 | 10,000 | 12,000 | 25,000 | 20,000 | 50,000 |
| CVC-sep 3 | 15,000 | 20,000 | 12,000 | 25,000 | 12,000 | 25,000 | 40,000 | 50,000 |
| Endocard 1 | 30,000 | 50,000 | 40,0007 | 5,000 | 20,000 | 50,000 | 20,000 | 100,000 |
| Endocard 2 | 15,000 | 12,000 | 7,000 | 5,000 | 6,000 | 12,000 | 20,000 | 50,000 |
| Endocard 3 | 12,000 | 6,000 | 4,000 | 80,000 | 4,000 | 4,000 | 7,000 | 50,000 |

| | | | | | | | | |
|---|---|---|---|---|---|---|---|---|
| * Titres are the serum dilutions required to reduce the absorbance at 450nm to 0.1 | | | | | | | | |

These results show that some strains contribute more antigen than others to the overall reactivity of BHI7 and that the ability of the ELISA to discriminate between positive and negative sera is best provided by strains 1-6.

### Example 5: Preparation of Antigen from Cultures Grown in Defined Medium

Chemical identification of the antigen present in BHI7 would have been complicated by the presence of material originating from the brain heart infusion medium in fractions 10-15 from the gel permeation chromatography column. The inventors therefore investigated the use of an alternative chemically defined medium (HHW) designed for growth of *S. epidermidis* (Hussain *et al*, 1992 J. Med. Microbiol. 37, 368-375).

The antigen was prepared in the same manner as for BHI7 using 6 strains (strain 7 omitted) grown in HHW, and this material was designated "HHW6". For the purposes of comparison, the inventors also purified the cellular LTA from whole cells of one of the 6 strains (strain 1) grown in HHW using a phenol extraction method (Coley *et al*, 1972 J. Gen. Micro. **73**, 587-591). LTA was coated onto the microtitre plates at a concentration of 0.05 mg/mL.

The performance of each preparation (BHI7, HHW6 and the purified LTA) in ELISA was compared on a set of 27 positive CVC-sepsis sera and 14 of the control sera. The sera were tested at a single dilution of 1:800 on each plate so that direct comparisons of the responses could be made. Each serum sample was applied to the plates before and after absorption with *Staph. epidermidis* LTA. Absorption was carried out by adding 50 µL of a 10 mg/mL solution of LTA to 2 mL of a 1:800 dilution of the sera in TTBS, incubating for 18 h at 4°C and centrifuging to remove anti-LTA antibody/LTA immune complexes. The statistical comparisons of the means of the CVC-sepsis and control groups is shown below in Tables 6 and 7.

**Table 6:**

| **Comparison of IgG ELISA mean absorbances for positive and negative CVC sepsis sera at 1:800 dilution** | | |
|---|---|---|
| **Antigen preparation used** **on ELISA plates** | **Mean (SD) CVC-sepsis; CVC, no** **infection** | **Mean (SD) CVC-sepsis; CVC, no** **infection after absorption with LTA** |
| BHI 7 | 0.4040 (0.1193); 0.1766 (0.0427) | 0.2859 (0.1058); 0.1357 (0.0299) |
| HHW 6 | 0.2760 (0.1026); 0.1303 (0.0366) | 0.1541 (0.0377); 0.1012 (0.0195) |
| LTA | 0.5631 (0.4802); 0.1272 (0.0450) | 0.0987 (0.0272); 0.0848 (0.0108) |

**Table 7:**

| **Statistical analysis (unpaired t-test) of ELISA results listed in Table 6** | | |
|---|---|---|
| **Antigen preparation used on** **ELISA plates** | **Difference in means, CVC-sepsis** **and CVC, no infection** | **Difference in means , CVC-sepsis and** **CVC, no infection, LTA-absorbed sera** |
| BHI7 | 0.2275, P<0.0001 extremely significant | 0.1502, P<0.0001 extremely significant |
| HHW6 | 0.1367, P<0.0001 extremely significant | 0.0529, P<0.0001 extremely significant |
| LTA | 0.4359, P<0.0001 extremely significant | 0.0139, P = 0.0259 significant |

The results show that the 3 different antigen preparations (BHI7, HHW6 and LTA) each gave a significant difference in IgG ELISA responses between the CVC-sepsis and control sera. The responses were considerably reduced following a single absorption with LTA. The reduction in absorbance in the assays was 70% for BHI7, 44% for HHW6 and 83% for the LTA. This suggested that LTA, or something antigenically related to LTA, was a major component of the BHI7 and HHW6 antigen preparations. Failure to absorb more antibody from the BHI7 and HHW6 preparations suggested that they contained additional antigenic determinants to those present on the purified LTA of the single strain.

The same methods were also used to prepare antigen preparations from each of the seven strains grown individually in HHW medium.

Each HHW antigen preparation described above was separately evaluated for its performance in ELISA using the standard assay conditions described previously for the BHI7 and HHW6 antigens. Fractions 10-15 from each elution profile were pooled separately, freeze dried to determine the dry weight and re-dissolved in carbonate buffer to 0.01mg/ml. Separate batches of ELISA plates were coated and used to examine sera from 50 patients with CVC-sepsis due to *S. epidermidis* and 50 controls. Commercially available preparations of LTA from *Staph. aureus,* and a laboratory preparation of cellular LTA from *Staph. epidermidis*, prepared by the method of Fischer *et al* (1983 Eur. J. Biochem. 133, 523-530), were included for comparison. The mean results are shown below in Table 8, (M-W.P. = Mann-Whitney test).

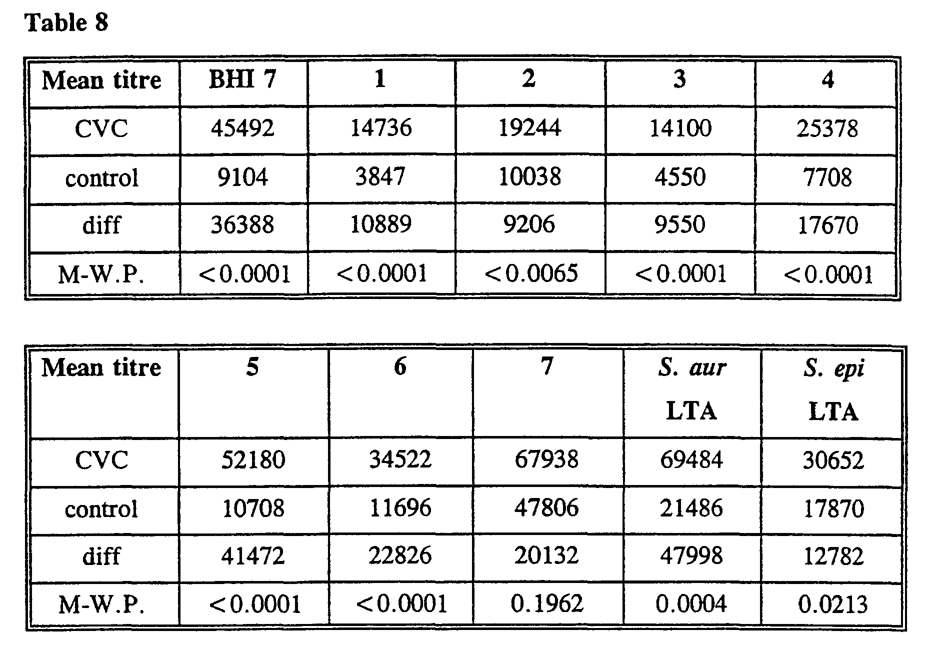

The results show that antigen prepared from strains 1-4 and strain 6, and BHI7, all perform better than commercially available *Staph. aureus* LTA and perform much better than LTA extracted from *Staph. epidermidis* cells. Subsequent ROC analysis of these data confirmed that the HHW antigen preparations performed better than commercially available *Staph. aureus* LTA when used to coat ELISA plates for diagnosis of CVC sepsis. BHI 7 antigen performance was better than that of any of the component strains when judged by ROC analysis.

### Example 6: Characterisation of the antigen

Indirect evidence from the effect of extracted LTA from *Staph. epidermidis* upon the BHI7 ELISA suggested that the antigen contained LTA or something antigenically related to LTA. No structural determination of the LTA of *Staph. epidermidis* has yet been reported, but the LTA of *Staph. aureus* mainly consists of 28 glycerol phosphate units linked to a glycolipid, as shown in Figure 1.

Confirmation by the direct chemical analysis of the BHI7 antigen could not be carried out because of the presence of contaminating levels of components derived from the brain heart infusion medium. Exocellular antigen was therefore prepared from the culture medium of strain 1 following growth in the chemically defined medium (HHW). The chemical nature of this material was investigated by negative electrospray mass spectrometry, ³¹P and ¹H NMR and chemical analysis of the acid hydrolysed material.

### Yield of antigen

The method for antigen preparation was exactly as described for the BHI7 antigen. Strain 1 was grown in 2 L of HHW at 37°C for 18 h on a rotary shaker. Culture medium was recovered by centrifugation, freeze dried and reconstituted in water to 10-times the original concentration. 1mL portions were applied to the Superose 12 column and eluted in water at 0.2 mL/min collecting 0.8 mL fractions. Fractions 10-15 were pooled and freeze dried. 45 mL of pooled fractions yielded 5.5 mg dry weight of material as a white powder, equivalent to 58.7 mg/L of medium. This material was subjected to a variety of analyses.

### Example 6.1: Physical Analysis

### Negative electrospray mass spectrometry

The position of elution of the antigen from the Superose 12 gel permeation column in fractions 10-15 immediately after the void volume (fraction 9) suggested that the material had a high molecular weight (estimated as > 100,000 Daltons from the elution of standard protein markers). Although conventional mass spectrometry only permits analysis of molecules with charge to mass ratios up to 1600, the presence of a number of negative charges (which would be present if the material were LTA) enables higher molecular weight molecules to be analysed. The electrospray technique does not break the molecule into fragments but detects the relative abundance of molecules with different mass/charge ratios.

Accordingly, 1 mg of the purified antigen was subjected to negative electrospray MS using a Hewlett Packard MS instrument (model 5989B) with a Hewlett Packard electrospray accessory unit (model 59987A) operating at 10 µL of sample per min. The sample (approximately 0.1 mg) was dissolved in 2 mL methanol/water (95/5 v/v) containing 50 µL ammonia (0.88 s.g.). The mass spectrum is shown in the lower frame of Figure 6A where the x axis records the fragment mass/negative charge ratio (m/z) and the y axis the abundance. Strong peaks were detected at m/z values of 804 and 1206.

As the difference between these fragments (402) was exactly divisible into each, the spectrum could be deconvoluted to give an accurate and unambiguous molecular weight of 2415.16 atomic mass units (Figure 6A upper frame). Furthermore, because major fragments of m/z 804 and 1206 were detected, those fragments must bear negative charges of -3 and -2 respectively and the intact molecule has a total charge of -6. This accurate determination of both the molecular mass and the total negative charge of the antigen was central to its structural determination. It indicated that, if the antigen was a molecule with a structure analogous to that of conventional *Staph. aureus* LTA, the glycerol phosphate chain length must be very much shorter (i.e. a total of 6 phosphate units, each with 1 negative charge).

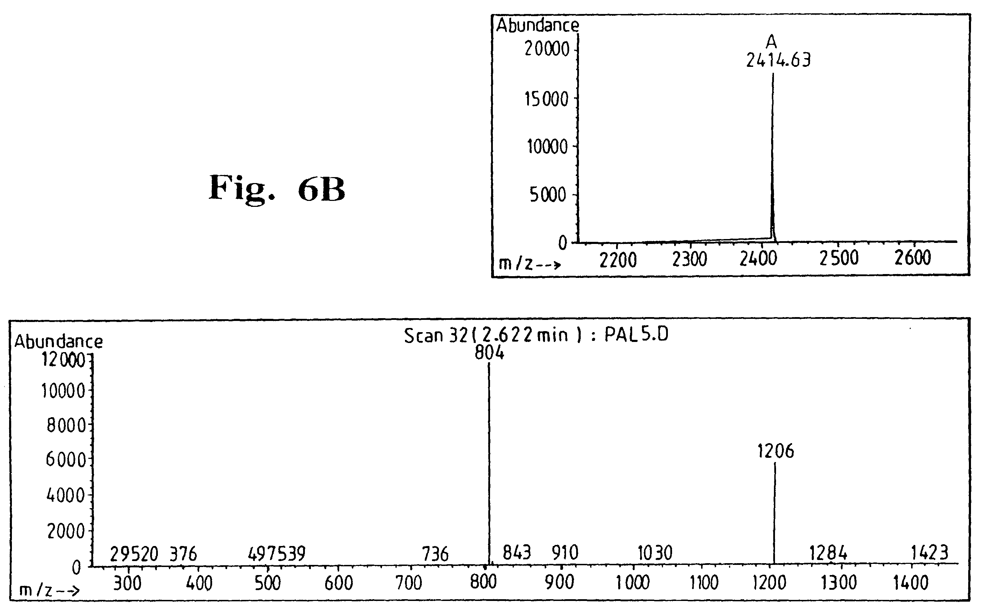

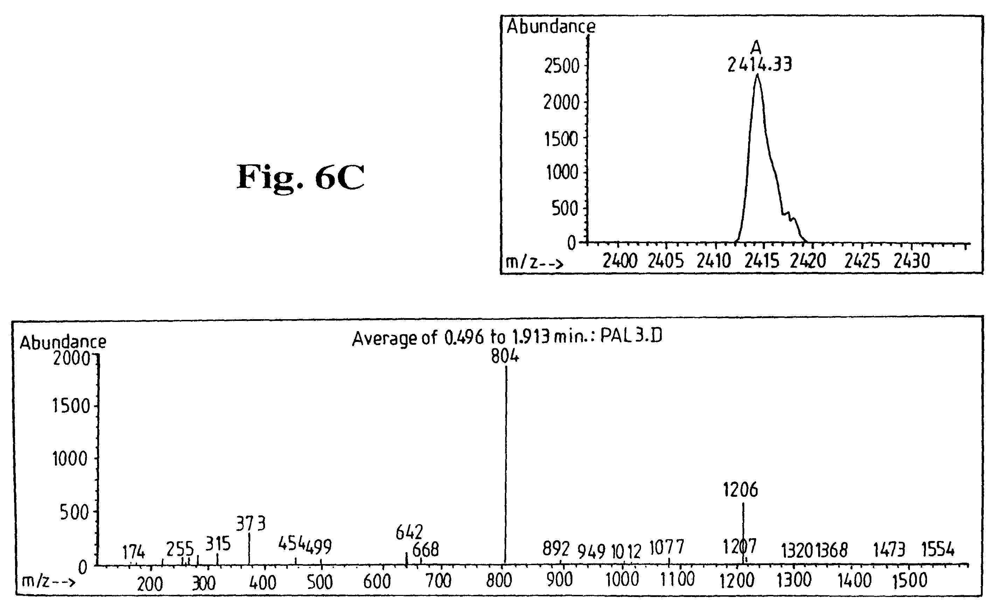

Subsequently, identical analysis was performed on antigen prepared from cultures of strain Nos. 3 and 4, and essentially identical results were obtained (Figures 6B and 6C).

### ³¹P and ¹H NMR spectra

Purified material was submitted for NMR analysis using a 250 MHz Bruker instrument operating at 20°C. The ³¹P spectrum was obtained using 2 mg of material dissolved in D₂O. The phosphorus chemical shift was measured as parts per million (ppm) relative to an internal reference of 80% phosphoric acid (data omitted for brevity).

Two major signals at 6.33 and 3.01 ppm were evident together with a number of smaller peaks. The spectrum confirmed the presence of phosphorus in the material and indicated that the phosphorus atoms occurred in a number of different magnetic environments.

Similar results have been reported for ³¹P spectra of LTA from a range of organisms (Batley *et al* 1987 Biochim. Biophys. Acta **901**, 127-137). The splitting of the peaks is thought to indicate high flexibility in the glycerol phosphate chains rather than different chemical linkages of the phosphates. One explanation is the variation in substituents on the 2-position of the glycerol units in the poly(glycerolphosphate) chain, phosphate esters on unsubstituted glycerols giving a signal at higher ppm than phosphates on glycerol units bearing alanine substituents.

The ¹H NMR spectrum was obtained using 2 mg of material dissolved in dimethyl sulphoxide (DMSO). The chemical shift of the signals in ppm was measured relative to the DMSO signal at 2.5 ppm (data omitted for brevity).

Signals obtained at 0.8 and 1.21 ppm were typical of aliphatic protons CH₃ and (CH₂)ₙ present in methylene chains and are similar to those reported for the fatty acid chains of the glycolipid moiety of LTA (Batley *et al*, 1987, cited above). Protons present on the glycerol moieties of LTA would appear in the region 3.94 - 4.12. Unfortunately this area contained major peaks at 3.459 ppm from contaminating water and at 2.489 ppm from DMSO.

### Example 6.2: Chemical analysis

The mass spectrum and NMR data of the antigen from strain 1 suggested an LTA structure analogous to that of *Staph. aureus* but with a much shorter glycerol phosphate chain length. Chemical analysis of the material was undertaken to confirm the presence of the structural components (i.e. fatty acids, glucose, glycerol and phosphate) and to determine the ratio of each component.

### Fatty acids

Fatty acid content was determined by alkaline methanolysis and gas-liquid chromatography (GC). 1 mg of material was suspended in 1 mL of 3.8 M NaOH in 50% aqueous methanol in a glass hydrolysis tube. The tube was sealed and incubated at 100°C for 30 mins then cooled to room temperature. Any fatty acids liberated were converted to the corresponding fatty acid methyl esters (FAMEs) by the addition of 6 mL of 6 M HCl/methanol (1:1) and heating at 80°C for 10 minutes. FAMEs were extracted with 1 ml hexane/diethyl ether (1:1). The upper solvent layer was removed and placed in a glass tube containing 3 mL of 0.3 M NaOH. After mixing by repeated inversion the organic phase was recovered and transferred to a 2 mL glass sample tube. The solvent was evaporated to dryness by the passage of nitrogen gas into the tube at room temperature.

For quantitative analysis by GC the sample was redissolved in 100 µL of hexane and 1 µL was loaded onto a Hewlett Packard HP-1 capillary column (crosslinked methyl silicone gum, ID 0.32 mm, film thickness 0.17 µm, length 25 m) on a Unicam 610 series GC operating with 1:50 sample splitting and flame ionisation detector. The column temperature was programmed to maintain 150°C for 4 min then increased at 4°C per min to 250°C and held at this temperature for 2 min. The gas phase comprised helium at 6.5 psi with nitrogen as the makeup gas. The flame ionisation detector used hydrogen and air, the injector temperature was set at 200°C and the detector temperature was set at 280°C. Fatty acids were identified and estimated quantitatively by comparison with the profile obtained for 1 µL of a standard bacterial FAME mix containing 26 bacterial FAMEs (Matreya Inc.) diluted in hexane to a concentration of 5 µg/µL. Integration readings were calculated for each peak and the total amount of fatty acid in the original sample calculated. The results are shown in Table 9.

**Table 9:**

| **Fatty acid composition of the purified antigen from strain 1 grown in HHW** | | |
|---|---|---|
| **Fatty acid** | **Retention time** **(min)** | **Relative amount** **(%)** |
| 13-methyltetradecanoate (iso-15:0) | 11.0 | 2.5 |
| 12-methyltetradecanoate (anteiso-15:0) | 11.2 | 37.8 |
| 15-methylhexadecanoate (iso-17:0) | 16.0 | 8.8 |
| cis-9-octadecanoate (18:1) | 18.3 | 0.8 |
| octadecanoate (18.0) | 19.0 | 4.5 |
| unidentified | 20.4 | 2.5 |
| cis-9,10-methyleneoctadecanoate (19:0 cycloprop) | 20.6 | 13.1 |
| eicosanoate (20:0) | 23.4 | 26.6 |
| unidentified | 25.0 | 3.4 |

The total amount of fatty acid present in the antigen was 35.9 µg/mg. Fatty acid analysis of the whole cell pellet of strain 1 grown in HHW gave a similar profile to that of the purified antigen showing that the fatty acids in the antigen are typical of those in the total lipids of the organism and are similar to those reported for *Staph. epidermidis* (Behme *et al*, 1996 J. Clin. Micro. **34**, 3075-3084).

### Acid hydrolysis of the antigen for analysis of hexose, phosphate and glycerol content

A 2.4 mg sample of the purified antigen was dissolved in 0.3 ml of 2M trifluoroacetic acid (TFA), the tube was sealed and heated at 100°C for 18 h. A white precipitate formed when the TFA was first added. After hydrolysis the TFA was removed by repeated drying under vacuum with addition of water. The hydrolysis products were then dissolved in 0.24 mL of water to give a concentration equivalent to 10 mg/mL of original antigen. Samples of this TFA hydrolysate were subjected to different analyses to determine the nature of hexoses present (as alditol acetates by GC) and the relative amounts of hexose, phosphate and glycerol by quantitative colourimetric assays. An empty hydrolysis tube was treated with TFA in the same way as the sample, this was used in all assays as a control (hydrolysis blank).

### Conversion of hexoses to alditol acetates and analysis by GC

The conversion of hexoses in the TFA hydrolysate to the alditol acetates and their identification by GC was carried out to determine which sugars were present (Takayama & Kilburn 1989 Antimicrobial Agents and Chemotherapy **33**, 1493-1499). The method did not yield accurate estimations of the total amount present because of losses in the conversion and recovery of the alditol acetates. 0.1 mL of the TFA hydrolysate (equivalent to 1mg of antigen) was placed in a glass tube with 50 µL of 3M NH₄OH. 3 mg of sodium borohydride was added and the solution left in the dark at 22°C for 18 h. 1 drop of glacial acetic acid was added to destroy excess borohydride. The borate was converted to the methyl derivative by addition of methanol (0.5 mL) and rotary evaporated to dryness at 50°C. Another portion of methanol (0.5 mL) was added and the sample again evaporated to dryness. Acetic anhydride (0.1 mL) was then added to the dried alditol sample, the tube sealed and heated in an autoclave at 121°C for 3h. Excess acetic anhydride was destroyed by adding water (0.4 mL) and the alditol acetates were purified by passage through a Sep-Pak C18 cartridge (Waters Associates Inc.). The cartridge was prepared by washing with acetonitrile (2 mL) followed by water (1 mL). The sample was loaded onto the cartridge, washed with 10% acetonitrile (2 mL) and eluted in 40% acetonitrile (2m L). The eluted alditol acetates were rotary evaporated to dryness, redissolved in chloroform (0.10 mL) and analysed by GC.

1 µL was loaded onto a Supelco SP-2380 capillary column (ID 0.25 mm, film thickness 0.2 µm, length 30 m) on a Unicam 610 series GC operating with 1:100 sample splitting and flame ionisation detector. The column temperature was maintained at 250°C. The gas phase comprised helium at 6.5 psi with nitrogen as the makeup gas (flow rate 25.05 cm/sec). The flame ionisation detector used hydrogen and air, the injector temperature was set at 200°C and the detector temperature was set at 280°C. A mixture (1 µL) of the alditol acetates of mannitol, galactitol, glucitol and inositol (Supelco, 5 mg/mL total in chloroform) was run as a standard giving retention times of 7.91, 8.66, 9.38 and 10.37 min respectively. The sample contained one major peak of retention time 9.383 min which was identified as glucitol. There were in addition 9 minor peaks detected on the chromatogram. However, an identical pattern of these minor peaks (but lacking the glucitol acetate peak at 9.38 min) was obtained by analysis of the hydrolysis blank processed in the same way for alditol acetate preparation. The antigen was therefore presumed to contain glucose as the major hexose component, but quantitative estimation was considered inaccurate.

### Hexose estimation by phenol sulphuric assay

Assay of the total hexose in the TFA hydrolysate was carried out using the phenol sulphuric acid reagent (Dubois *et al*, 1956 cited above). Measured volumes of a standard glucose solution (1 µg/µL) were placed in glass tubes to give a range of 0 to 50 µg of glucose per tube, the volumes were then adjusted to 0.2 mL with water. 10 µL and 20 µL samples of the TFA hydrolysate and blank hydrolysate were placed in separate tubes and the volumes also made up to 0.2 ml with water. 50 µL of an 8% w/v aqueous phenol solution as added to each tube followed by 0.5 ml of concentrated sulphuric acid. The tubes were allowed to stand for 10 min then transferred to a water bath at 30°C for 20 min. The absorbance was then measured at 492 nm and the amount of hexose (as glucose) present in the sample was calculated from the standard glucose calibration curve. 20 µL of the TFA sample hydrolysate contained 16.7 µg of glucose, equivalent to 83.5 µg of glucose per mg of antigen.

### Phosphorus assay

Phosphorus present in the sample TFA hydrolysate was measured as phosphate after treatment with alkaline phosphatase to release phosphate from any remaining glycerol phosphate residues (Ames 1966 Methods in Enzymology **8**, 115-118). 0.1mL samples of the TFA hydrolysate and the hydrolysis blank were treated with 10 µL of an aqueous solution (1 mg/mL) of alkaline phosphatase (calf intestinal phosphomonoesterase, Sigma) for 2 h at room temperature. 5 µL and 10 µL samples were then placed in glass tubes, separate tubes containing 1 µL to 5 µg of phosphorus from a standard solution containing 10 µg/mL phosphorus (prepared by making a stock solution of 87.8 mg of KH₂PO₄ in 100 mL water and diluting 5 mL of this solution to 100 mL with water). Colour reagent was prepared by mixing 3 M sulphuric acid (10 mL) with 2.5% w/v ammonium molybdate (10 mL) and adding to 1 g of ascorbic acid. 1 mL of the colour reagent was added to each tube and incubated at 37°C for 1.5 h. The absorbance was read at 750 nm and the total phosphorus content of the sample calculated from the standard curve. 10 µL of TFA hydrolysate contained 3 µg of phosphorus, equivalent to 30 µg of phosphorus per mg of antigen.

### Glycerol assay

The glycerol content of the TFA hydrolysate was determined by periodate oxidation and measurement of the formaldehyde released with chromotropic acid (Ames 1966, cited above). 0.1 mL of glycerol standard containing 2 µg to 20 µg glycerol, 10 µL of TFA hydrolysate or blank hydrolysate were placed in separate glass tubes. 20 µL of concentrated sulphuric acid was added followed by 20 µL of 0.1 M aqueous sodium periodate. The tubes were allowed to stand at room temperature for 5 min. 20 µL of 10% w/v aqueous sodium bisulphite was added followed by 0.5 mL of chromotropic acid solution (containing 0.1 g of chromotropic acid in 10 mL of water and 45 mL of concentrated sulphuric acid). The tubes were heated at 100°C for 30 min, cooled and 50µL of saturated aqueous thiourea added. The absorbance was then measured at 570 nm. The amount of glycerol in the TFA hydrolysate was calculated from the standard curve. 10 µL of TFA hydrolysate contained 6.4 µg of glycerol, equivalent to 67 µg glycerol per mg of antigen.

### Ratio of constituents of the antigen

Combining the results of the chemical analyses gave the following ratios:

| **glycerol** | **phosphorus** | **fatty acid** | **glucose** |
|---|---|---|---|
| 67 µg/mg | 32 µg/mg | 36 µg/mg | 84 µg/mg |
| 0.73 µmol/mg | 1.0 µmol/mg | 0.31 µmol/mg* | 0.46 µmol/mg |

| | | | |
|---|---|---|---|
| *assuming an average chain length from Table 10 | | | |

This in turn suggests a molar ratio of:
5 glycerol units: 7 phosphorus (as phosphate): 2 fatty acids: 3 glucose

Despite the errors likely in the analyses (estimated as being +/- 10% based on volume and weight measurements) and the possible loss of material during TFA hydrolysis, these results strongly support the presence in the antigen of a short chain LTA molecule comprising a glycolipid (e.g. diacylgentiobiosylglycerol) with a glycerol phosphate chain length of 6 units (analogous to conventional long chain LTA). The chemical analysis does not account for all of the material present in the antigen on a weight basis. This is due partly to the losses during hydrolysis but also to the presence of other components of the LTA which were not analysed (e.g. alanyl esters or N-acetylglucosamine substituents on the glycerol phosphate chain). The negative electrospray mass spectrum gave an accurate mass of 2415 with 6 negative charges. The molecular mass based on chemical analysis would be 1892:

**Table 10**

| **Component** | **number of mols** | **mol wt of unit** | **wt in antigen** |
|---|---|---|---|
| glycerol | 7* | 74 | 444 |
| phosphate | 6 | 80 | 480 |
| glucose | 3 | 162 | 486 |
| fatty acid | 2 | 241 | 482 |
| Total molecular weight | | | 1892 |

| | | | |
|---|---|---|---|
| *assuming 1 glycerol also present as diglyceride, so that 6 glycerol residues are in the chain. | | | |

Therefore additional mass units of 723 (2415-1892) need to be found to account for the molecular weight of 2415. The two most likely candidate components not yet analysed (N-acetylglucosamine and D-alanine) have masses of 203 and 61 respectively. With 6 potential glycerol units available for substitution a combination of these additional components might account for the extra molecular weight. The behaviour of the antigen as a high molecular weight species on gel permeation chromatography is explained by the formation of micelles at concentration above 5µM (Wicken *et al*, 1986 J. Bact. **166**, 72-77).

### Example 7: Optimised ELISA Protocol

The inventors have devised a standardised protocol for the preparation of BHI7 antigen, and a rapid, optimised ELISA protocol for sero-diagnostic detection of Gram+ve infections. The details of these protocols are set out below.

### Protocol for preparation of the antigen:

The 7 strains of *Staphylococcus epidermidis* and related organisms are maintained on separate brain heart infusion plates (Oxoid). These are stored at 4°C, subculturing at 4 week intervals onto fresh plates. The plates are checked for culture purity by colonial appearance and Gram staining after each subculture.
1. Prepare separate starter cultures for each strain by inoculating 2-3 colonies from the respective maintenance plates into 20mL of brain heart infusion broth (Oxoid) in 100mL conical flasks. Incubate the broth cultures for 18h at 37°C on a rotary shaker (200rpm).
2. Inoculate 1ml of each starter culture into separate 500mL conical flasks containing 200mL of brain heart infusion (use a separate culture flask for each strain). Incubate the flasks for 18h at 37°C on a rotary shaker (200rpm).
3. Check each culture for purity by streaking onto brain heart infusion plates, incubate for 18h at 37°C, observe colonial appearance and Gram-stain typical colonies. [The results of this culture purity check will be available whilst the following stages are in progress. The batch of purified antigen should only be passed for use if all of the cultures are pure].
4. Centrifuge each of the 200ml cultures (10,000g, 10 min) and pool the supernatant fluids (1400mL total volume). Freeze the pooled supernatant fluid in suitable freeze drying vessels (e.g. 100mL portions in 500mL flasks) and remove the water by freeze drying. This stage can be carried out in separate batches, depending on the capacity of the freeze drying apparatus. Batches of frozen pooled culture medium can be stored at -20°C before freeze drying.
5. Dissolve the freeze dried material in water to one tenth of the original volume (e.g. 10mL for each 100mL of pooled culture medium). Store this concentrated pooled culture medium in separate 1.5mL portions (e.g. in 1.5mL conical plastic Eppendorf tubes) at -20°C prior to the following gel permeation stage.
6. Thaw one Eppendorf tube of the frozen concentrated culture medium, warm to 20°C to dissolve as much of the insoluble material as possible, centrifuge in a plastic conical tube for 2 min at 13,500g in a microfuge to deposit any remaining insoluble material.
7. Apply 1ml of the supernatant fluid to a Superose 12 prepacked HR 10/30 FPLC column (Pharmacia) equilibrated with water. Elute with water at 0.4mL/min, collecting 50 fractions of 0.8mL each. Pool fractions 10-15 inclusive and store at -20°C until needed to coat the ELISA plates. 1mL of this purified material is sufficient to coat 10 separate 96-well ELISA plates. It should be stored frozen in appropriate volumes (e.g. 1mL) according to the number of plates to be coated per batch.

### Protocol for coating and blocking ELISA plates

1. Thaw a tube of frozen purified antigen from stage 7 in the antigen preparation protocol (e.g. 1mL) and dilute with 100 volumes (e.g. 100mL) of sodium carbonate/bicarbonate buffer (0.05M, pH 9.6). Place 0.1mL of this diluted antigen into each well of 96-well polystyrene microtitre plates (Immulon 2, Dynatech) and leave covered with a sheet of aluminium foil at 4°C for 18h.
2. Remove the antigen from the wells by rapidly inverting and shaking the plate over a sink. Gently tap the empty inverted plate on a pad of dry paper towels to remove all traces of the liquid. Return the plate to the correct orientation and immerse the entire plate in a suitable container filled with TBS-Tween solution (0.01M Tris-HCl pH 7.4, NaCl 0.9% w/v, 0.3% v/v Tween-20). Remove the plate from the container ensuring that every well is completely filled with solution. Immediately remove the TBS-Tween solution by inverting the plate over the sink and repeat the process of filling the wells with TBS-Tween by immersion in the container three further times to remove all traces of residual unbound antigen. Cover the filled plate with aluminium foil and leave for 1h at 4°C.
3. Remove the TBS-Tween solution from the wells by inverting the plate and gently tap the empty inverted plate on a pad of dry paper towels to remove all traces of the liquid. Seal the antigen-coated, blocked plates in a labelled polythene bag and store at -20°C until required for an assay.

### Protocol for ELISA:

1. Remove a coated, blocked ELISA plate from the freezer (stage 3 of the protocol for coating and blocking plates) and allow to reach room temperature.
2. Prepare 1:6400 dilutions of the positive and negative control sera and each patient serum to be tested in TBS-Tween solution (0.01M Tris-HCl pH 7.4, NaCl 0.9% w/v, 0.3% v/v Tween-20).
3. Place 0.1mL of the diluted sera (in duplicate) into wells of the ELISA plate.
4. Cover the plate with aluminium foil and leave for 2h at 37°C.
5. Remove the serum dilutions from the wells by rapidly inverting and shaking the plate over a sink. Gently tap the empty inverted plate on a pad of dry paper towels to remove all traces of the liquid. Return the plate to the correct orientation and immerse the entire plate in a suitable container filled with TBS-Tween solution (0.01M Tris-HCl pH 7.4, NaCl 0.9% w/v, 0.3% v/v Tween-20). Remove the plate from the container ensuring that every well is completely filled with solution. Immediately remove the TBS-Tween solution by inverting the plate over the sink and repeat the process of filling the wells with TBS-Tween by immersion in the container three further times to remove all traces of residual unbound antigen. Gently tap the empty inverted plate on a pad of dry paper towels to remove all traces of the liquid. [This manual plate washing procedure may be replaced by a suitable washing protocol using an automated plate washer with TBS-Tween solution].
6. Prepare a solution of the chosen conjugate reagent in TBS-Tween: either 0.5 µg/mL protein A-horseradish peroxidase conjugate (Sigma) or goat anti-human IgG-peroxidase (Sigma) at the manufacturer's recommended dilution for ELISA.
7. Place 0.1mL of the chosen diluted conjugate solution in all wells on the plate. Cover with aluminium foil and leave for 30min (protein A-peroxidase) or 1h (antihuman-IgG-peroxidase) at 37°C.
8. Remove the conjugate solution from the wells on the plate and wash with TBS-Tween solution as described in 5 above.
9. Prepare a solution of the chromogenic peroxidase substrate substrate by dissolving 10mg of 3,3'5,5'-tetramethylbenzidine (Sigma) in 1 mL of dimethyl sulphoxide and dilute with 100 mL of sodium acetate/citrate buffer (0.1M, pH 6.0) containing 10 µL of H₂O₂ (20% v/v). Ensure that the solution is at 37°C. Place 0. 1mL of this solution into each well on the plate. Allow the blue colour to develop for 25 min and then stop the reaction by addition of 100µL of sulphuric acid (1M) toeach well. This produces a stable yellow solution which should be measured within 1h with a plate reader.
10. Measure the absorbance of the yellow coloured product in each well at 450nm with a suitable plate reader (e.g. Anthos 2001, Anthos Labtec Instruments).
11. Subtract the mean absorbance value of the negative control from that of the positive control and each of the test samples. Calculate the ratio of the absorbances for each test sample to that of the positive control (which has a titre defined as 100,000) and multiply the ratio by 100,000. This gives the standardised titre for each sample.

The serum dilution of 1:6,400 has been found to give good discrimination between sera from infected or uninfected patients (in the case of endocarditis or CVC-sepsis). A similar dilution should also be suitable in testing sera from patients with prosthetic hip replacements, although slight variation (e.g. +/- 10-15%) in the dilution factor may be desired for optimum results.

The use of a single dilution avoids the need for serial serum dilutions across the ELISA plate. This makes the optimised ELISA far quicker to perform, and enables more sera to be tested on a single ELISA plate.

As an alternative to the titre calculation described above, it may be desired to compare the O.D. of the test sera with a calibration curve of O.D. provided by known dilutions of a positive control serum.

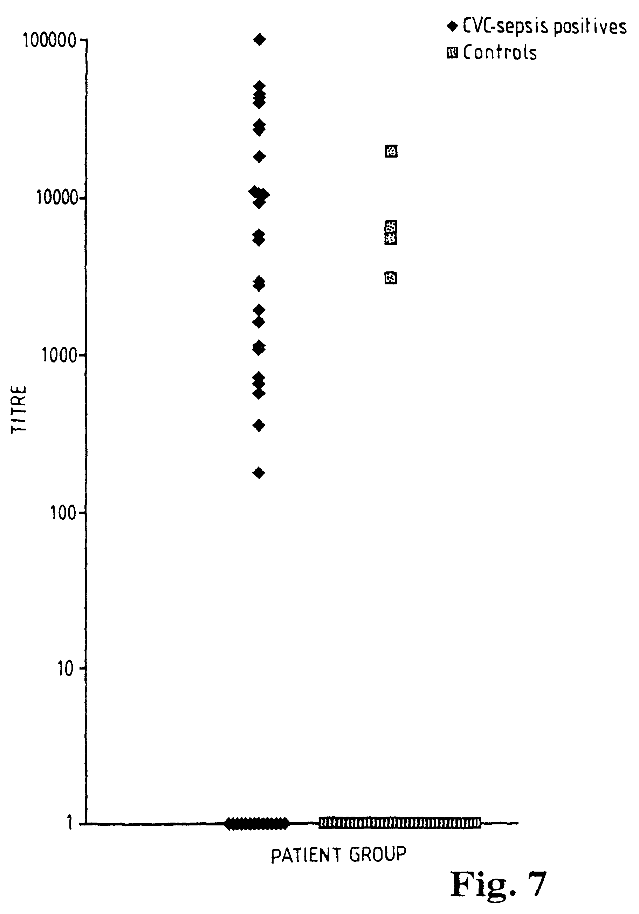

The optimised ELISA protocol decribed above was used to test sera from patients with CVC-sepsis or sera from a control group of patients. The data are shown in the scattergraph in Figure 7. The results are extremely clear cut. The mean titre for positive sera (n=40) was 10,429, whilst that for control sera (n=40) was 865 (p < 0.0001). It will be noted that four control sera had high titres. These were probably due to the presence of a minor, undiagnosed Gram positive infection (e.g. local skin infection) or due to a recent unreported exposure to a Gram positive organism. Low titres in some of the test sera are probably explained by the health of the individuals, many of whom were immunocompromised or immuno suppressed as a result of their condition and/or treatment.

## Claims

1. An isolated compound having the structure 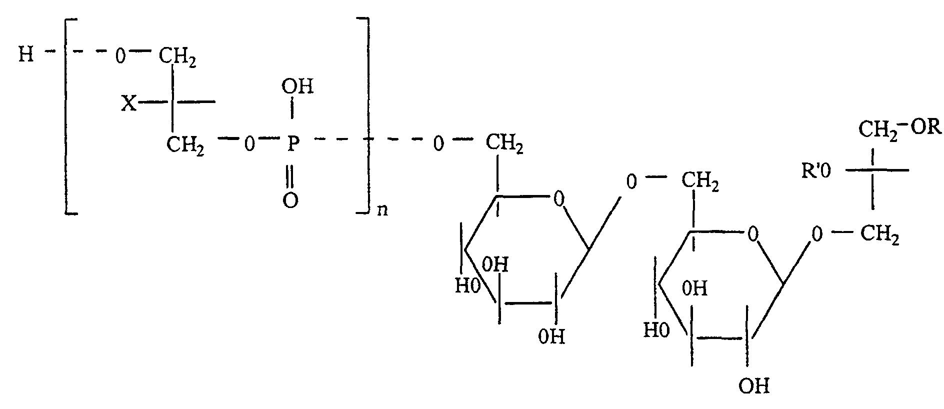 wherein n is an integer between 3 and 10 inclusive and X is H, OH, alkyl, aryl, amyl, or an amino acid residue, optionally substituted, or a sugar residue, optionally substituted, and wherein R and R¹ are hydrophobic hydrocarbon or fatty acid chains and wherein R may be the same as R¹ or different, and wherein the value of n in any single sample is a single integer, other than a compound wherein n is 3 and X is OH.

2. A compound according to claim 1, wherein n = 6.

3. A compound according to claim 1 or 2, wherein X = H, OH, D-alanyl or N-acetyl glucosamine.

4. A composition in which, of the bacterial components present, at least 50 w/w % comprises a compound having the structure 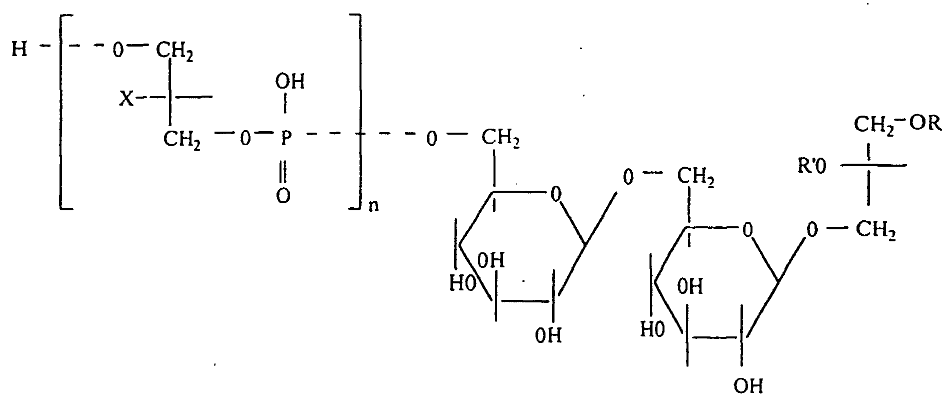 wherein n is an integer between 3 and 10 inclusive and X is H, OH, alkyl, aryl, amyl, or an amino acid residue, optionally substituted, or a sugar residue, optionally substituted, and wherein R and R¹ are hydrophobic hydrocarbon or fatty acid chains and wherein R may be the same as R¹ or different, other than a compound wherein n is 3 and X is OH.

5. A composition according to claim 4, in the form of a freeze-dried solid, an aqueous solution, or immobilised on a solid support.

6. A method of testing for a Gram +ve bacterial infection in a mammalian, typically human subject, the method comprising the steps of: contacting a sample of body fluid from the subject with a composition comprising a compound having the structure 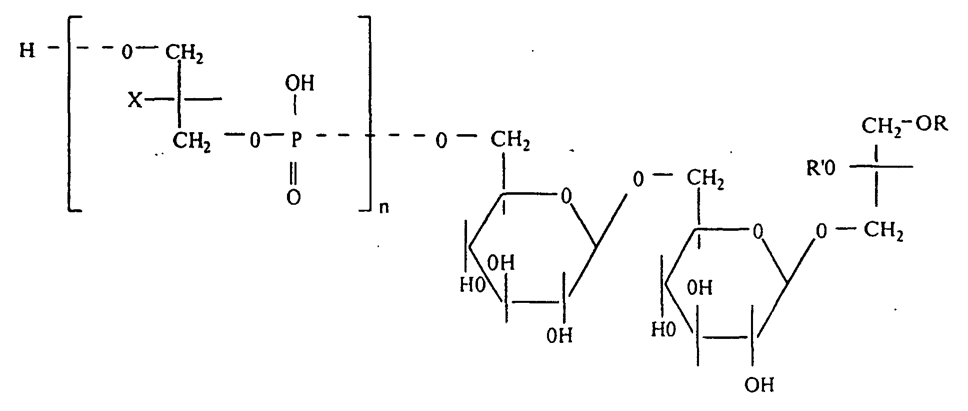 wherein n is an integer between 3 and 10 inclusive and X is H, OH, alkyl, aryl, amyl, or an amino acid residue, optionally substituted, or a sugar residue optionally substituted, and R and R¹ are hydrophobic hydrocarbon or fatty acid chains wherein R may be the same as R¹, or different; and detecting binding of antibodies, if any, in the sample to the composition.

7. A method according to claim 6, wherein the sample of body fluid obtained from the subject comprises whole blood, serum, urine or saliva.

8. A method according to claim 6 or 7, comprising the detection of binding to the composition of IgG antibodies in the sample.

9. A method according to any one of claims 6, 7 or 8, wherein the test method comprises the performance of an enzyme-linked immunosorbent assay (ELISA), radioimmunoassay (RIA), or a Western blot.

10. A method according to any one of claims 6-9, for testing for infection caused by Gram +ve cocci.

11. A method according to any one of claims 6-10, for testing for infection by a Streptococcus, a Staphylococcus or an Enterococcus.

12. A method according to any one of claims 6 to 11, for diagnosing the presence of a Gram +ve infection associated with a central venous catheter, a cerebrospinal fluid shunt or a prosthetic device.

13. A method according to any one of claims 6 to 12, wherein the composition is in accordance with claim 4 or 5.

14. A diagnostic test kit for diagnosing the presence of a Gram +ve infection in a mammalian subject, the kit comprising: a solid support for performing a diagnostic test; and a composition in accordance with claim 4 or 5.

15. A kit according to claim 14, further comprising one or more of the following: labelled antibody; enzyme substrate; control sample; buffer; and instructions for use.

16. A sterile vaccine composition for use against a Gram +ve infection in a mammalian subject, the vaccine comprising an isolated compound in accordance with any one of claims 1 to 3, or a composition in accordance with claim 4 or 5.

17. An isolated immunoglobulin molecule or variant thereof having specific binding for a compound in accordance with any one of claims 1 to 3.

18. An isolated eukaryotic cell producing an immunoglobulin molecule or variant thereof in accordance with claim 17.

19. A method of making a composition in accordance with claim 4 or 5, the method comprising the steps of: culturing a Gram +ve bacterium in a growth medium so as to cause the bacterium to secrete into the growth medium a compound in accordance with any one of claims 1-3; separating the growth medium from the bacterial cells; fractionating the growth medium; and isolating that fraction which comprises, in substantially pure form, the compound in accordance with any one of claims 1-3.

20. *Staphylococcus epidermidis* strain CAN 6KIII, deposited under accession number NCIMB 40896.

21. *Staphylococcus epidermidis* strain HAR 6KIV, deposited under accession number NCIMB 40945.

22. *Staphylococcus epidermidis* strain COS 6KV, deposited under accession number NCIMB 40946.

23. *Staphylococcus epidermidis* strain MIL 6LI, deposited under accession number NCIMB 40947.

24. *Staphylococcus epidermidis* strain HED 6LI, deposited under accession number NCIMB 40948.

25. *Staphylococcus haemolyticus* strain ONE 6KVI, deposited under accession number NCIMB 40949.

26. *Micrococcus kristinae* strain MAT 6LII, deposited under accession number NCIMB 40950.

27. A method according to claim 19, comprising the step of culturing one or more organisms selected from the group consisting of: *Staphylococcus epidermidis* strain CAN 6KIII; *Staphylococcus epidermidis* strain HAR 6KIV; *Staphylococcus epidermidis* strain COS 6KV; *Staphylococcus epidermidis* strain MIL 6LI; *Staphylococcus epidermidis* strain HED 6LI; *Staphylococcus haemolyticus* strain ONE 6KVI; *Micrococcus kristinae* strain MAT 6LII.

28. A method of obtaining an immunoglobulin or antigen-binding variant thereof having specific binding for a compound in accordance with any one of claims 1-3, the method comprising the steps of: screening a library of viruses or other particles displaying an immunoglobulin or antigen-binding variant thereof on their surface; and selecting those members of the library which display an immunoglobulin or antigen-binding variant thereof which bind to the compound.

29. A vaccine for inducing antibodies in a mammalian subject the vaccine comprising a composition in accordance with claim 5 and a physiologically acceptable excipient, carrier or diluent.

30. Use of an isolated immunoglobulin having specific binding activity for a compound in accordance with any one of claims 1-3 in the preparation of a medicament to provide passive immunisation of a mammalian subject for the prevention and/or treatment of a Gram +ve bacterial infection.

## Patentansprüche

1. Isolierte Verbindung mit der Struktur 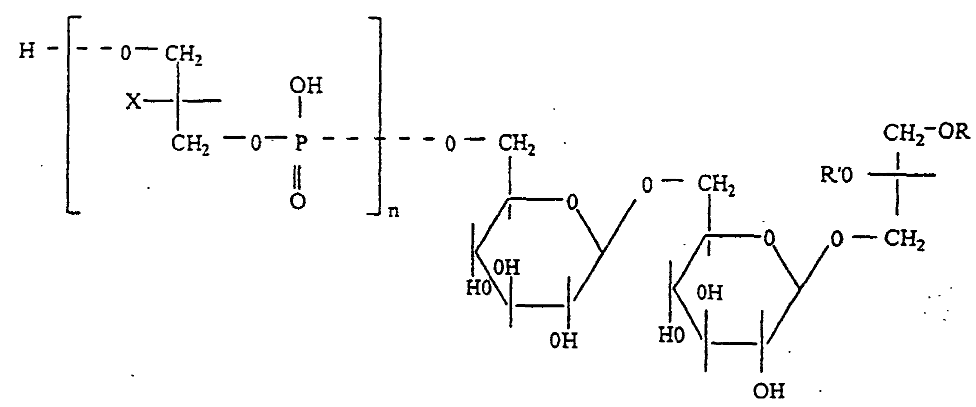 wobei n eine ganze Zahl von einschließlich 3 bis einschließlich 10 ist und X H, OH, Alkyl, Aryl, Amyl oder ein Aminosäurerest ist, fakultativ substituiert, oder ein Zuckerrest, fakultativ substituiert, und wobei R und R¹ hydrophobe Kohlenwasserstoff- oder Fettsäureketten sind und wobei R gleich R¹ oder dazu unterschiedlich sein kann, und wobei der Wert von n in einer einzelnen Probe eine einzelne ganze Zahl ist, wobei die Verbindung nicht eine Verbindung ist, in der n 3 und X OH ist.

2. Verbindung gemäß Anspruch 1, wobei n = 6 ist.

3. Verbindung gemäß Anspruch 1 oder 2, wobei X = H, OH, D-Alanyl oder N-Acetylglucosamin ist.

4. Zusammensetzung, in der in Bezug auf die vorliegenden bakteriellen Komponenten mindestens 50 Gew.-% von einer Verbindung mit der Struktur 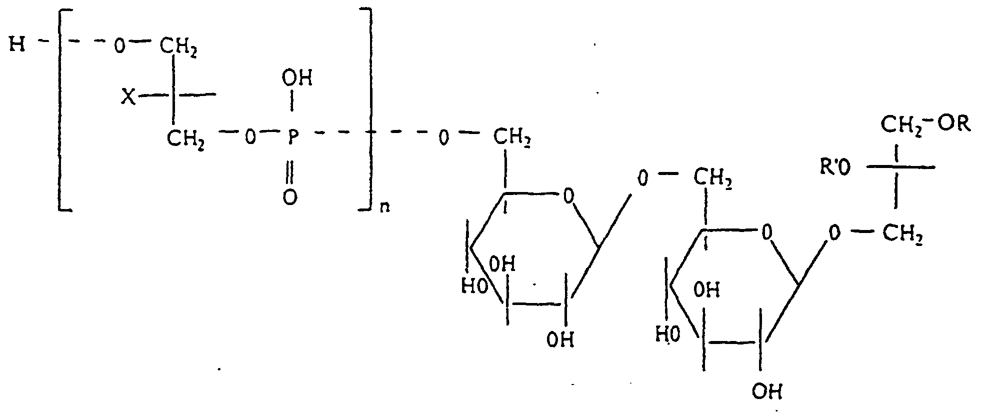 umfaßt wird, wobei n eine ganze Zahl von einschließlich 3 bis einschließlich 10 ist und X H, OH, Alkyl, Aryl, Amyl oder ein Aminosäurerest ist, fakultativ substituiert, oder ein Zuckerrest, fakultativ substituiert, und wobei R und R¹ hydrophobe Kohlenwasserstoffoder Fettsäureketten sind und wobei R gleich R¹ oder dazu unterschiedlich sein kann, wobei die Verbindung nicht eine Verbindung ist, in der n 3 und X OH ist.

5. Zusammensetzung gemäß Anspruch 4 in Form eines gefriergetrockneten Feststoffes, einer wäßrigen Lösung oder immobilisiert auf einem festen Träger.

6. Verfahren zum Testen auf eine Infektion durch Gram positive Bakterien in einem Säugetier, typischerweise in einem menschlichen Individuum, wobei das Verfahren folgende Schritte umfaßt: in Kontakt bringen einer Probe einer Körperflüssigkeit aus dem Individuum mit einer Zusammensetzung, umfassend eine Verbindung mit der Struktur 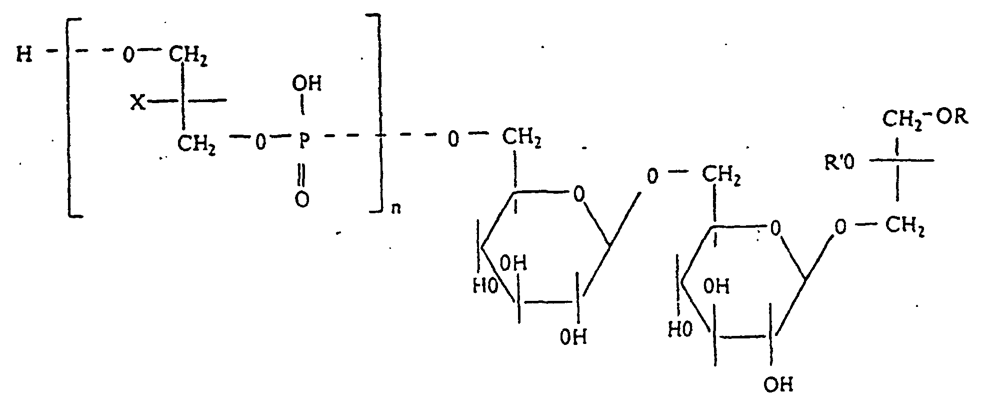 wobei n eine ganze Zahl von einschließlich 3 bis einschließlich 10 ist und X H, OH, Alkyl, Aryl, Amyl oder ein Aminosäurerest ist, fakultativ substituiert, oder ein Zuckerrest, fakultativ substituiert, und wobei R und R¹ hydrophobe Kohlenwasserstoff- oder Fettsäureketten sind, wobei R gleich R¹ oder dazu unterschiedlich sein kann; und Nachweisen der Bindung von Antikörpern, falls vorhanden, in der Probe an die Zusammensetzung.

7. Verfahren gemäß Anspruch 6, wobei die Probe der Körperflüssigkeit, die von dem Individuum erhalten wurde, Vollblut, Serum, Urin oder Speichel umfaßt.

8. Verfahren gemäß Anspruch 6 oder 7, umfassend den Nachweis der Bindung von IgG-Antikörpem in der Probe an die Zusammensetzung.

9. Verfahren gemäß mindestens einem der Ansprüche 6, 7 oder 8, wobei das Testverfahren die Durchführung eines enzymgebundenen immunosorbenten Assays (ELISA), einen Radioimmunoassay (RIA) oder einen Western-Blot umfaßt.

10. Verfahren gemäß mindestens einem der Ansprüche 6 bis 9 zum Testen auf eine Infektion, die von Gram positiven Kokken verursacht wird.

11. Verfahren gemäß mindestens einem der Ansprüche 6 bis 10 zum Testen auf eine Infektion, die von einem Streptokokkus, einem Staphylokokkus oder einem Enterokokkus verursacht wird.

12. Verfahren gemäß mindestens einem der Ansprüche 6 bis 11 zur Diagnose des Vorliegens einer Gram positiven Infektion, die mit einem zentralen venösen Katheter, einem cerebrospinalen Flüssigkeitsnebenschluß oder einer prothetischen Vorrichtung verbunden ist.

13. Verfahren gemäß mindestens einem der Ansprüche 6 bis 12, wobei die Zusammensetzung eine solche gemäß Anspruch 4 oder 5 ist.

14. Diagnostischer Testkit zur Diagnose auf das Vorliegen einer Gram positiven Infektion in einem Säugetierindividuum, wobei der Kit umfaßt: einen festen Träger zum Durchführen eines diagnostischen Tests und eine Zusammensetzung gemäß Anspruch 4 oder 5.

15. Kit gemäß Anspruch 14, der darüber hinaus eines oder mehrere der folgenden umfaßt: markierter Antikörper; Enzymsubstrat; Kontrollprobe; Puffer und Gebrauchsanweisung.

16. Sterile Impfzusammensetzung zur Verwendung gegen eine Gram positive Infektion in einem Säugetierindividuum, wobei der Impfstoff eine isolierte Verbindung gemäß mindestens einem der Ansprüche 1 bis 3 umfaßt oder eine Zusammensetzung gemäß Anspruch 4 oder 5.

17. Isoliertes Immunoglobulinmolekül oder eine Variante davon, die spezifisch an eine Verbindung gemäß mindestens einem der Ansprüche 1 bis 3 bindet.

18. Isolierte eukaryotische Zelle, die ein Immunoglobulinmolekül oder eine Variante davon gemäß Anspruch 17 produziert.

19. Verfahren zur Herstellung einer Zusammensetzung gemäß Anspruch 4 oder 5, wobei das Verfahren folgende Schritte umfaßt: Kultivierung eines Gram positiven Bakteriums in einem Wachstumsmedium, um die Sekretion einer Verbindung gemäß mindestens einem der Ansprüche 1 bis 3 durch das Bakterium in das Wachstumsmedium hervorzurufen; Separieren des Wachstumsmediums von den bakteriellen Zellen; Fraktionierung des Wachstumsmediums und Isolieren der Fraktion, die, in im wesentlichen reiner Form, die Verbindung gemäß mindestens einem der Ansprüche 1 bis 3 umfaßt.

20. *Staphylokokkus epidermis*-Stamm CAN 6KIII, hinterlegt unter der Zugangsnummer NCIMB 40896.

21. *Staphylokokkus epidermis*-Stamm HAR 6KIV, hinterlegt unter der Zugangsnummer NCIMB 40945.

22. *Staphylokokkus epidermis*-Stamm COS 6KV, hinterlegt unter der Zugangsnummer NCIMB 40946.

23. *Staphylokokkus epidermis*-Stamm MIL 6LI, hinterlegt unter der Zugangsnummer NCIMB 40947.

24. *Staphylokokkus epidermis*-Stamm HED 6LI, hinterlegt unter der Zugangsnummer NCIMB 40948.

25. *Staphylokokkus haemolyticus*-Stamm ONE 6KVI, hinterlegt unter der Zugangsnummer NCIMB 40949.

26. *Microkokkus kristinae*-Stamm MAT 6LII, hinterlegt unter der Zugangsnummer NCIMB 40950.

27. Verfahren gemäß Anspruch 19, umfassend den Schritt der Kultivierung voneinem oder mehreren Organismen, ausgewählt aus der Gruppe, die besteht aus: *Staphylokokkus epidermis*-Stamm CAN 6KIII; *Staphylokokkus epidermis*-Stamm HAR 6KIV; *Staphylokokkus epidermis*-Stamm COS 6KV; *Staphylokokkus epidermis*-Stamm MIL 6LI; *Staphylokokkus epidermis*-Stamm HED 6LI; *Staphylokokkus haemolyticus*-Stamm ONE 6KVI; *Microkokkus kristinae*-Stamm MAT 6LII.

28. Verfahren zum Erhalten eines Immunoglobulins oder einer Antigen-bindenden Variante davon mit spezifischer Bindung an eine Verbindung gemäß mindestens einem der Ansprüche 1 bis 3, wobei das Verfahren folgende Schritte umfaßt: Screenen einer Bank von Viren oder anderen Partikeln, die ein Immunoglobulin oder eine Antigen-bindende Variante davon auf ihrer Oberfläche präsentieren; und Selektieren derjenigen Bestandteile der Bibliothek, die ein Immunoglobulin oder eine Antigen-bindende Variante davon, die an die Verbindung binden, präsentieren.

29. Impfstoff zum Induzieren von Antikörpern in einem Säugetierindividuum, wobei der Impfstoff eine Zusammensetzung gemäß Anspruch 5 und einen physiologisch verträglichen Hilfsstoff, Träger oder Verdünner umfaßt.

30. Verwendung eines isolierten Immunoglobulins mit spezifischer Bindungsaktivität für eine Verbindung gemäß mindestens einem der Ansprüche 1 bis 3 zur Herstellung eines Medikaments, um einem Säugetierindividuum passive Immunisierung zu verleihen, um eine Gram positive bakterielle Infektion zu verhindern und/oder zu behandeln.

## Revendications

1. Composé isolé ayant la structure 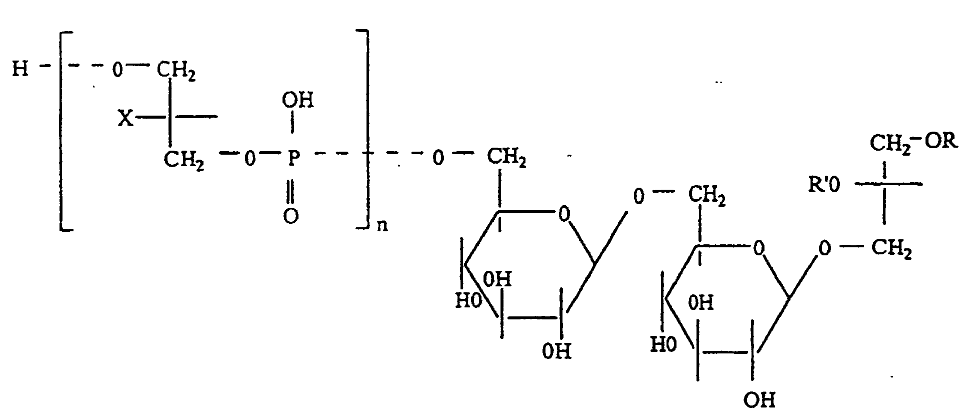 dans laquelle n est un nombre entier entre 3 et 10 inclus et X est H, OH, alkyle, aryle, amyle, ou un reste d'acide aminé, éventuellement substitué, ou un reste de sucre, éventuellement substitué, et dans laquelle R et R¹ sont des chaînes hydrocarbonées hydrophobes ou d'acide gras et dans laquelle R peut être identique ou différent de R¹, et dans laquelle la valeur de n dans n'importe quel échantillon unique est un entier unique, à l'exception d'un composé dans lequel n est 3 et X est OH.

2. Composé selon la revendication 1, dans lequel n = 6.

3. Composé selon la revendication 1 ou 2, dans lequel X = H, OH, D-alanyle ou N-acéthylglucosamine.

4. Composition dans laquelle, parmi les composants bactériens présents, au moins 50 % (p/p) comprend un composé ayant la structure 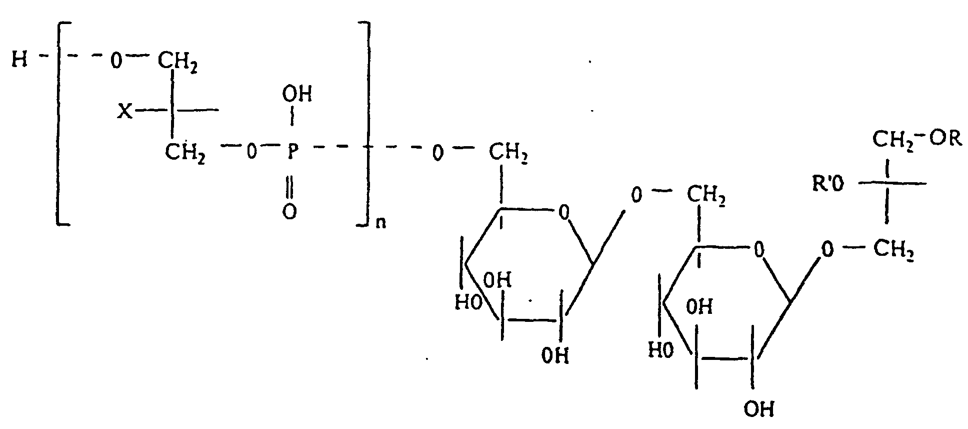 dans laquelle n est un nombre entier entre 3 et 10 inclus et X est H, OH, alkyle, aryle, amyle, ou un reste d'acide aminé, éventuellement substitué, ou un reste de sucre, éventuellement substitué, et dans laquelle R et R¹ sont des chaînes hydrocarbonées hydrophobes ou d'acide gras et dans laquelle R peut être identique ou différent de R¹, à l'exception d'un composé dans lequel n est 3 et X est OH.

5. Composition selon la revendication 4, sous forme d'un solide lyophilisé, une solution aqueuse, ou immobilisée sur un support solide.

6. Méthode d'analyse d'une infection bactérienne à Gram +ve chez un mammifère, typiquement un sujet humain, la méthode comprenant les étapes consistant à : mettre en contact un échantillon d'un liquide corporel du sujet avec une composition comprenant un composé ayant la structure 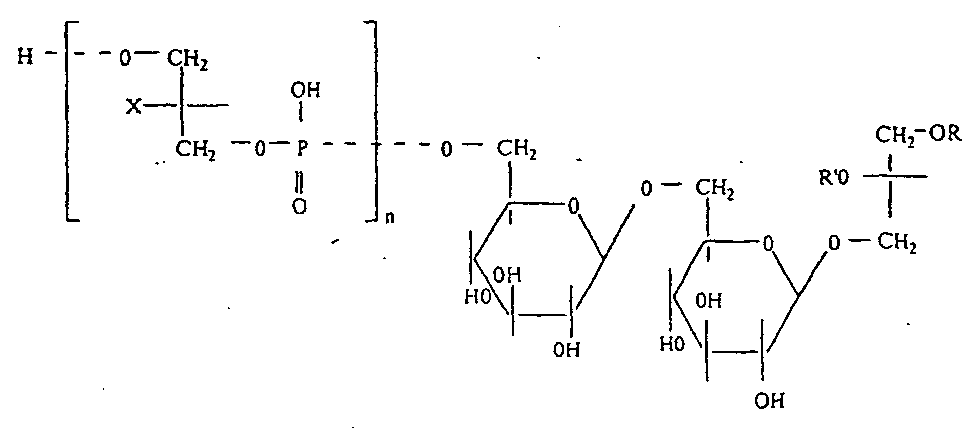 dans laquelle n est un nombre entier entre 3 et 10 inclus et X et H, OH, alkyle, aryle, amyle, ou un reste d'acide aminé, éventuellement substitué, ou un reste de sucre, éventuellement substitué, et dans laquelle R et R¹ sont des chaînes hydrocarbonées hydrophobes ou d'acide gras et dans laquelle R peut être identique à R¹ ou différent ; et la détection de la liaison d'anticorps, le cas échéant, à la composition dans l'échantillon.

7. Méthode selon la revendication 6, dans laquelle l'échantillon de liquide corporel obtenu d'un sujet comprend du sang total, du sérum, de l'urine ou de la salive.

8. Méthode selon la revendication 6 ou 7, comprenant la détection de la liaison à la composition d'anticorps IgG dans l'échantillon.

9. Méthode selon l'une quelconque des revendications 6, 7 ou 8, dans laquelle la méthode d'analyse comprend la réalisation d'un essai par immuno-sorbant lié à une enzyme (ELISA), un essai radio-immunologique (RIA), ou un Western blot.

10. Méthode selon l'une quelconque des revendications 6 à 9, pour l'analyse d'une infection causée par des coques Gram +ve.

11. Méthode selon l'une quelconque des revendications 6 à 10, pour l'analyse d'une infection par un streptocoque, un staphylocoque ou un entérocoque.

12. Méthode selon l'une quelconque des revendications 6 à 11, pour le diagnostique de la présence d'une infection à Gram +ve associée à un cathéter veineux central, un circuit de dérivation du fluide cérébrospinal ou un dispositif prothétique.

13. Méthode selon l'une quelconque des revendications 6 à 12, dans laquelle la composition est selon la revendication 4 ou 5.

14. Kit pour test de diagnostic pour le diagnostic de la présence d'une infection à Gram +ve chez un mammifère, le kit comprenant : un support solide pour la réalisation d'un test de diagnostic; et une composition selon la revendication 4 ou 5.

15. Kit selon la revendication 14, comprenant en outre l'un ou plusieurs des suivants : un anticorps marqué, un substrat enzymatique, un échantillon témoin, un tampon, et des instructions pour son utilisation.

16. Composition vaccinale stérile pour une utilisation contre une infection à Gram +ve chez un mammifère, le vaccin comprenant un composé isolé selon l'une quelconque des revendications 1 à 3, ou une composition selon la revendication 4 ou 5.

17. Molécule d'immunoglobuline isolée ou variant de celle-ci ayant une spécificité de liaison pour un composé selon l'une quelconque des revendications 1 à 3.

18. Cellule eucaryote isolée produisant une molécule d'immunoglobuline ou un variant de celle-ci selon la revendication 17.

19. Méthode de préparation d'une composition selon la revendication 4 ou 5, la méthode comprenant les étapes consistant à : cultiver une bactérie Gram +ve dans un milieu de croissance de manière à faire sécréter à la bactérie dans le milieu de culture un composé selon l'une quelconque des revendications 1 à 3 ; la séparation du milieu de culture des cellules bactériennes ; le fractionnement du milieu de culture; et l'isolement de la fraction qui comprend, sous forme essentiellement pure, le composé selon l'une quelconque des revendications 1 à 3.

20. *Staphylococcus epidermidis* souche CAN 6KIII, déposée sous le numéro d'accès NCIMB 40896.

21. *Staphylococcus epidermidis* souche HAR 6KIV, déposée sous le numéro d'accès NCIMB 40945.

22. *Staphylococcus epidermidis* souche COS 6KV, déposée sous le numéro d'accès NCIMB 40946.

23. *Staphylococcus epidermidis* souche MIL 6LI, déposée sous le numéro d'accès NCIMB 40947.

24. *Staphylococcus epidermidis* souche HED 6LI, déposée sous le numéro d'accès NCIMB 40948.

25. *Staphylococcus haemolyticus* souche ONE 6KVI, déposée sous le numéro d'accès NCIMB 40949.

26. *Micrococcus kristinae* souche MAT 6LII, déposée sous le numéro d'accès NCIMB 40950.

27. Méthode selon la revendication 19 comprenant l'étape de culture d'un ou plusieurs organismes choisis dans le groupe consistant en : *Staphylococcus epidermidis* souche CAN 6KIII ; *Staphylococcus epidermidis* souche HAR 6KIV ; *Staphylococcus epidermidis* souche COS 6KV ; *Staphylococcus epidermidis* souche MIL 6LI ; *Staphylococcus epidermidis* souche HED 6LI ; *Staphylococcus haemolyticus* souche ONE 6KVI ; *Micrococcus kristinae* souche MAT 6LII.

28. Méthode d'obtention d'une immunoglobuline ou d'un variant de celle-ci liant un antigène ayant une spécificité de liaison pour un composé selon l'une quelconque des revendications 1 à 3, la méthode comprenant les étapes consistant à : cribler une banque de virus ou d'autres particules présentant une immunoglobuline ou un variant de celle-ci liant un antigène à leur surface ; et la sélection des membres de la banque qui présentent une immunoglobuline ou un variant de celle-ci liant un antigène, qui se lient au composé.

29. Vaccin pour induire des anticorps chez un mammifère, le vaccin comprenant une composition selon la revendication 5 et un excipient, véhicule ou diluant physiologiquement acceptable.

30. Utilisation d'une immunoglobuline isolée ayant une activité de liaison spécifique pour un composé selon l'une quelconque des revendications 1 à 3 pour la préparation d'un médicament destiné à fournir une immunisation passive d'un mammifère pour la prévention et/ou le traitement d'une infection bactérienne à Gram +ve.
